# EUROPEAN PATENT SPECIFICATION

(11) **EP 1 541 685 B1**
(45) Date of publication and mention of the grant of the patent: **26.10.2011**
(21) Application number: 03799071.0
(22) Date of filing: 25.07.2003
(51) Int. Cl.: C12N 15/12, C12Q 1/68

(54) **FACTOR PARTICIPATING IN TRANSCRIPTIONAL REGULATION**
AN DER TRANSKRIPTIONSREGULATION BETEILIGTER FAKTOR
FACTEUR PARTICIPANT A LA REGULATION DE LA TRANSCRIPTION

(30) Priority: 25.07.2002 JP 2002217233
(43) Date of publication of application: 15.06.2005
(73) Proprietor: Sony Corporation, Tokyo 141-0001 (JP)
(72) Inventor: MIZUTANI, Shuki, Matsudo-shi, Chiba 270-2241 (JP); YAMADA, Takayuki, Kamagaya-shi, Chiba 273-0105 (JP)
(74) Representative: Horner, David Richard
(86) International application number: PCT/JP2003/009443
(87) International publication number: WO 2004/031388

(56) References cited:
- WO-A1-01/60855
- WO-A2-00/58473
- WO-A2-01/07471
- WO-A2-01/64834
- WO-A2-99/58559
- US-A1- 2005 069 986
- SMITH REBECCA L ET AL: "The tetratricopeptide repeats of Ssn6 interact with the homeo domain of alpha-2" GENES AND DEVELOPMENT, vol. 9, no. 23, 1995, pages 2903-2910, XP009064526 ISSN: 0890-9369
- LAMB J R ET AL: "Tetratrico peptide repeat interactions: to TPR or not to TPR?" TRENDS IN BIOCHEMICAL SCIENCES, ELSEVIER, HAYWARDS, GB, vol. 20, no. 7, July 1995 (1995-07), pages 257-259, XP004222275 ISSN: 0968-0004
- NAKATSU, Y ET AL: 'XAB2, a Novel Tetratricopeptide Repeat Protein Involved in Transcription-coupled DNA Repair and Transcription.' THE JOURNAL OF BIOLOGICAL CHEMISTRY vol. 275, no. 45, 2000, pages 34931 - 34937, XP002975852

### SEQUENCE LISTING

<110> MIZUTANI, Shuki
   YAMADA, Takayuki
<120> Transcription regulating factors
<130> SEN-AOI22P
<140>
   <141>
<150> JP 2002-217233
   <151> 2002-07-25
<160> 2
<170> PatentIn Ver. 2.0
<210> 1
   <211> 2684
   <212> DNA
   <213> Homo sapiens
<220>
   <221> CDS
   <222> (37)..(2601)
<400> 1
   agcgcgcgac tctcctgtac ctgggcatcc agaaaa atg gtg gtg atg gcg cga 54
   Met Val Val Met Ala Arg
   1 5
   ctt tcg cgg ccc gag cgg ccg gac ctt gtc ttc gag gaa gag gac ctc 102
   Leu Ser Arg Pro Glu Arg Pro Asp Leu Val Phe Glu Glu Glu Asp Leu
   10 15 20
   ccc tat gag gag gaa atc atg cgg aac caa ttc tct gtc aaa tgc tgg 150
   Pro Tyr Glu Glu Glu Ile Met Arg Asn Gln Phe Ser Val Lys Cys Trp
   25 30 35
   ctt cgc tac atc gag ttc aaa cag ggc gcc ccg aag ccc agg ctc aat 198
   Leu Arg Tyr Ile Glu Phe Lys Gln Gly Ala Pro Lys Pro Arg Leu Asn
   40 45 50
   cag cta tac gag cgg gca ctc aag ctg ctg ccc tgc agc tac aaa ctc 246
   Gln Leu Tyr Glu Arg Ala Leu Lys Leu Leu Pro Cys Ser Tyr Lys Leu
   55 60 65 70
   tgg tac cga tac ctg aag gcg cgt cgg gca cag gtg aag cat cgc tgt 294
   Trp Tyr Arg Tyr Leu Lys Ala Arg Arg Ala Gln Val Lys His Arg Cys
   75 80 85
   gtg acc gac cct gcc tat gaa gat gtc aac aac tgt cat gag agg gcc 342
   Val Thr Asp Pro Ala Tyr Glu Asp Val Asn Asn Cys His Glu Arg Ala
   90 95 100
   ttt gtg ttc atg cac aag atg cct cgt ctg tgg cta gat tac tgc cag 390
   Phe Val Phe Met His Lys Met Pro Arg Leu Trp Leu Asp Tyr Cys Gln
   105 110 115
   ttc ctc atg gac cag ggg cgc gtc aca cac acc cgc cgc acc ttc gac 438
   Phe Leu Met Asp Gln Gly Arg Val Thr His Thr Arg Arg Thr Phe Asp
   120 125 130
   cgt gcc ctc cgg gca ctg ccc atc acg cag cac tct cga att tgg ccc 486
   Arg Ala Leu Arg Ala Leu Pro Ile Thr Gln His Ser Arg Ile Trp Pro
   135 140 145 150
   ctg tat ctg cgc ttc ctg cgc tca cac cca ctg cct gag aca gct gtg 534
   Leu Tyr Leu Arg Phe Leu Arg Ser His Pro Leu Pro Glu Thr Ala Val
   155 160 165
   cga ggc tat cgg cgc ttc ctc aag ctg agt cct gag agt gca gag gag 582
   Arg Gly Tyr Arg Arg Phe Leu Lys Leu Ser Pro Glu Ser Ala Glu Glu
   170 175 180
   tac att gag tac ctc aag tca agt gac cgg ctg gat gag gcc gcc cag 630
   Tyr Ile Glu Tyr Leu Lys Ser Ser Asp Arg Leu Asp Glu Ala Ala Gln
   185 190 195
   cgc ctg gcc acc gtg gtg aac gac gag cgt ttc gtg tct aag gcc ggc 678
   Arg Leu Ala Thr Val Val Asn Asp Glu Arg Phe Val Ser Lys Ala Gly
   200 205 210
   aag tcc aac tac cag ctg tgg cac gag ctg tgc gac ctc atc tcc cag 726
   Lys Ser Asn Tyr Gln Leu Trp His Glu Leu Cys Asp Leu Ile Ser Gln
   215 220 225 230
   aat ccg gac aag gta cag tcc ctc aat gtg gac gcc atc atc cgc ggg 774
   Asn Pro Asp Lys Val Gln Ser Leu Asn Val Asp Ala Ile Ile Arg Gly
   235 240 245
   ggc ctc acc cgc ttc acc gac cag ctg ggc aag ctc tgg tgt tct ctc 822
   Gly Leu Thr Arg Phe Thr Asp Gln Leu Gly Lys Leu Trp Cys Ser Leu
   250 255 260
   gcc gac tac tac atc cgc agc ggc cat ttc gag aag gct cgg gac gtg 870
   Ala Asp Tyr Tyr Ile Arg Ser Gly His Phe Glu Lys Ala Arg Asp Val
   265 270 275
   tac gag gag gcc atc cgg aca gtg atg acc gtg cgg gac ttc aca cag 918
   Tyr Glu Glu Ala Ile Arg Thr Val Met Thr Val Arg Asp Phe Thr Gln
   280 285 290
   gtg ttt gac agc tac gcc cag ttc gag gag agc atg atc gct gca aag 966
   Val Phe Asp Ser Tyr Ala Gln Phe Glu Glu Ser Met Ile Ala Ala Lys
   295 300 305 310
   atg gag acc gcc tcg gag ctg ggg cgc gag gag gag gat gat gtg gac 1014
   Met Glu Thr Ala Ser Glu Leu Gly Arg Glu Glu Glu Asp Asp Val Asp
   315 320 325
   ctg gag ctg cgc ctg gcc cgc ttc gag cag ctc atc agc cgg cgg ccc 1062
   Leu Glu Leu Arg Leu Ala Arg Phe Glu Gln Leu Ile Ser Arg Arg Pro
   330 335 340
   ctg ctc ctc aac agc gtc ttg ctg cgc caa aac cca cac cac gtg cac 1110
   Leu Leu Leu Asn Ser Val Leu Leu Arg Gln Asn Pro His His Val His
   345 350 355
   gag tgg cac aag cgt gtc gcc ctg cac cag ggc cgc ccc cgg gag atc 1158
   Glu Trp His Lys Arg Val Ala Leu His Gln Gly Arg Pro Arg Glu Ile
   360 365 370
   atc aac acc tac aca gag gct gtg cag acg gtg gac ccc ttc aag gcc 1206
   Ile Asn Thr Tyr Thr Glu Ala Val Gln Thr Val Asp Pro Phe Lys Ala
   375 380 385 390
   aca ggc aag ccc cac act ctg tgg gtg gcg ttt gcc aag ttt tat gag 1254
   Thr Gly Lys Pro His Thr Leu Trp Val Ala Phe Ala Lys Phe Tyr Glu
   395 400 405
   gac aac gga cag ctg gac gat gcc cgt gtc atc ctg gag aag gcc acc 1302
   Asp Asn Gly Gln Leu Asp Asp Ala Arg Val Ile Leu Glu Lys Ala Thr
   410 415 420
   aag gtg aac ttc aag cag gtg gat gac ctg gca agc gtg tgg tgt cag 1350
   Lys Val Asn Phe Lys Gln Val Asp Asp Leu Ala Ser Val Trp Cys Gln
   425 430 435
   tgc gga gag ctg gag ctc cga cac gag aac tac gat gag gcc ttg cgg 1398
   Cys Gly Glu Leu Glu Leu Arg His Glu Asn Tyr Asp Glu Ala Leu Arg
   440 445 450
   ctg ctg cga aag gcc acg gcg ctg cct gcc cgc cgg gcc gag tac ttt 1446
   Leu Leu Arg Lys Ala Thr Ala Leu Pro Ala Arg Arg Ala Glu Tyr Phe
   455 460 465 470
   gat ggt tca gag ccc gtg cag aac cgc gtg tac aag tca ctg aag gtc 1494
   Asp Gly Ser Glu Pro Val Gln Asn Arg Val Tyr Lys Ser Leu Lys Val
   475 480 485
   tgg tcc atg ctc gcc gac ctg gag gag agc ctc ggc acc ttc cag tcc 1542
   Trp Ser Met Leu Ala Asp Leu Glu Glu Ser Leu Gly Thr Phe Gln Ser
   490 495 500
   acc aag gcc gtg tac gac cgc atc ctg gac ctg cgt atc gca aca ccc 1590
   Thr Lys Ala Val Tyr Asp Arg Ile Leu Asp Leu Arg Ile Ala Thr Pro
   505 510 515
   cag atc gtc atc aac tat gcc atg ttc ctg gag gag cac aag tac ttc 1638
   Gln Ile Val Ile Asn Tyr Ala Met Phe Leu Glu Glu His Lys Tyr Phe
   520 525 530
   gag gag agc ttc aag gcg tac gag cgc ggc atc tcg ctg ttc aag tgg 1686
   Glu Glu Ser Phe Lys Ala Tyr Glu Arg Gly Ile Ser Leu Phe Lys Trp
   535 540 545 550
   ccc aac gtg tcc gac atc tgg agc acc tac ctg acc aaa ttc att gcc 1734
   Pro Asn Val Ser Asp Ile Trp Ser Thr Tyr Leu Thr Lys Phe Ile Ala
   555 560 565
   cgc tat ggg ggc cgc aag ctg gag cgg gca cgg gac ctg ttt gaa cag 1782
   Arg Tyr Gly Gly Arg Lys Leu Glu Arg Ala Arg Asp Leu Phe Glu Gln
   570 575 580
   gct ctg gac ggc tgc ccc cca aaa tat gcc aag acc ttg tac ctg ctg 1830
   Ala Leu Asp Gly Cys Pro Pro Lys Tyr Ala Lys Thr Leu Tyr Leu Leu
   585 590 595
   tac gca cag ctg gag gag gag tgg ggc ctg gcc cgg cat gcc atg gcc 1878
   Tyr Ala Gln Leu Glu Glu Glu Trp Gly Leu Ala Arg His Ala Met Ala
   600 605 610
   gtg tac gag cgt gcc acc agg gcc gtg gag ccc gcc cag cag tat gac 1926
   Val Tyr Glu Arg Ala Thr Arg Ala Val Glu Pro Ala Gln Gln Tyr Asp
   615 620 625 630
   atg ttc aac atc tac atc aag cgg gcg gcc gag atc tat ggg gtc acc 1974
   Met Phe Asn Ile Tyr Ile Lys Arg Ala Ala Glu Ile Tyr Gly Val Thr
   635 640 645
   cac acc cgc ggc atc tac cag aag gcc att gag gtg ctg tcg gac gag 2022
   His Thr Arg Gly Ile Tyr Gln Lys Ala Ile Glu Val Leu Ser Asp Glu
   650 655 660
   cac gcg cgt gag atg tgc ctg cgg ttt gca gac atg gag tgc aag ctc 2070
   His Ala Arg Glu Met Cys Leu Arg Phe Ala Asp Met Glu Cys Lys Leu
   665 670 675
   ggg gag att gac cgc gcc cgg gcc atc tac agc ttc tgc tcc cag atc 2118
   Gly Glu Ile Asp Arg Ala Arg Ala Ile Tyr Ser Phe Cys Ser Gln Ile
   680 685 690
   tgt gac ccc cgg acg acc ggc gcg ttc tgg cag acg tgg aag gac ttt 2166
   Cys Asp Pro Arg Thr Thr Gly Ala Phe Trp Gln Thr Trp Lys Asp Phe
   695 700 705 710
   gag gtc cgg cat ggc aat gag gac acc atc aag gaa atg ctg cgt atc 2214
   Glu Val Arg His Gly Asn Glu Asp Thr Ile Lys Glu Met Leu Arg Ile
   715 720 725
   cgg cgc agc gtg cag gcc acg tac aac acg cag gtc aac ttc atg gcc 2262
   Arg Arg Ser Val Gln Ala Thr Tyr Asn Thr Gln Val Asn Phe Met Ala
   730 735 740
   tcg cag atg ctc aag gtc tcg ggc agt gcc acg ggc acc gtg tct gac 2310
   Ser Gln Met Leu Lys Val Ser Gly Ser Ala Thr Gly Thr Val Ser Asp
   745 750 755
   ctg gcc cct ggg cag agt ggc atg gac gac atg aag ctg ctg gaa cag 2358
   Leu Ala Pro Gly Gln Ser Gly Met Asp Asp Met Lys Leu Leu Glu Gln
   760 765 770
   cgg gca gag cag ctg gcg gct gag gcg gag cgt gac cag ccc ttg cgc 2406
   Arg Ala Glu Gln Leu Ala Ala Glu Ala Glu Arg Asp Gln Pro Leu Arg
   775 780 785 790
   gcc cag agc aag atc ctg ttc gtg agg agt gac gcc tcc cgg gag gag 2454
   Ala Gln Ser Lys Ile Leu Phe Val Arg Ser Asp Ala Ser Arg Glu Glu
   795 800 805
   ctg gca gag ctg gca cag cag gtc aac ccc gag gag atc cag ctg ggc 2502
   Leu Ala Glu Leu Ala Gln Gln Val Asn Pro Glu Glu Ile Gln Leu Gly
   810 815 820
   gag gac gag gac gag gac gag atg gac ctg gag ccc aac gag gtt cgg 2550
   Glu Asp Glu Asp Glu Asp Glu Met Asp Leu Glu Pro Asn Glu Val Arg
   825 830 835
   ctg gag cag cag agc gtg cca gcc gca gtg ttt ggg agc ctg aag gaa 2598
   Leu Glu Gln Gln Ser Val Pro Ala Ala Val Phe Gly Ser Leu Lys Glu
   840 845 850
   gac tgacccgtcc ctcccccatc ccccctcccc accccctccc caatacagct       2651
   Asp
   855
   acgtttgtac aaaaaaaaaa aaaaaaaaaa aaa 2684
<210> 2
   <211> 855
   <212> PRT
   <213> Homo sapiens
<400> 2
   Met Val Val Met Ala Arg Leu Ser Arg Pro Glu Arg Pro Asp Leu Val
   1 5 10 15
   Phe Glu Glu Glu Asp Leu Pro Tyr Glu Glu Glu Ile Met Arg Asn Gln
   20 25 30
   Phe Ser Val Lys Cys Trp Leu Arg Tyr Ile Glu Phe Lys Gln Gly Ala
   35 40 45
   Pro Lys Pro Arg Leu Asn Gln Leu Tyr Glu Arg Ala Leu Lys Leu Leu
   50 55 60
   Pro Cys Ser Tyr Lys Leu Trp Tyr Arg Tyr Leu Lys Ala Arg Arg Ala
   65 70 75 80
   Gln Val Lys His Arg Cys Val Thr Asp Pro Ala Tyr Glu Asp Val Asn
   85 90 95
   Asn Cys His Glu Arg Ala Phe Val Phe Met His Lys Met Pro Arg Leu
   100 105 110
   Trp Leu Asp Tyr Cys Gln Phe Leu Met Asp Gln Gly Arg Val Thr His
   115 120 125
   Thr Arg Arg Thr Phe Asp Arg Ala Leu Arg Ala Leu Pro Ile Thr Gln
   130 135 140
   His Ser Arg Ile Trp Pro Leu Tyr Leu Arg Phe Leu Arg Ser His Pro
   145 150 155 160
   Leu Pro Glu Thr Ala Val Arg Gly Tyr Arg Arg Phe Leu Lys Leu Ser
   165 170 175
   Pro Glu Ser Ala Glu Glu Tyr Ile Glu Tyr Leu Lys Ser Ser Asp Arg
   180 185 190
   Leu Asp Glu Ala Ala Gln Arg Leu Ala Thr Val Val Asn Asp Glu Arg
   195 200 205
   Phe Val Ser Lys Ala Gly Lys Ser Asn Tyr Gln Leu Trp His Glu Leu
   210 215 220
   Cys Asp Leu Ile Ser Gln Asn Pro Asp Lys Val Gln Ser Leu Asn Val
   225 230 235 240
   Asp Ala Ile Ile Arg Gly Gly Leu Thr Arg Phe Thr Asp Gln Leu Gly
   245 250 255
   Lys Leu Trp Cys Ser Leu Ala Asp Tyr Tyr Ile Arg Ser Gly His Phe
   260 265 270
   Glu Lys Ala Arg Asp Val Tyr Glu Glu Ala Ile Arg Thr Val Met Thr
   275 280 285
   Val Arg Asp Phe Thr Gln Val Phe Asp Ser Tyr Ala Gln Phe Glu Glu
   290 295 300
   Ser Met Ile Ala Ala Lys Met Glu Thr Ala Ser Glu Leu Gly Arg Glu
   305 310 315 320
   Glu Glu Asp Asp Val Asp Leu Glu Leu Arg Leu Ala Arg Phe Glu Gln
   325 330 335
   Leu Ile Ser Arg Arg Pro Leu Leu Leu Asn Ser Val Leu Leu Arg Gln
   340 345 350
   Asn Pro His His Val His Glu Trp His Lys Arg Val Ala Leu His Gln
   355 360 365
   Gly Arg Pro Arg Glu Ile Ile Asn Thr Tyr Thr Glu Ala Val Gln Thr
   370 375 380
   Val Asp Pro Phe Lys Ala Thr Gly Lys Pro His Thr Leu Trp Val Ala
   385 390 395 400
   Phe Ala Lys Phe Tyr Glu Asp Asn Gly Gln Leu Asp Asp Ala Arg Val
   405 410 415
   Ile Leu Glu Lys Ala Thr Lys Val Asn Phe Lys Gln Val Asp Asp Leu
   420 425 430
   Ala Ser Val Trp Cys Gln Cys Gly Glu Leu Glu Leu Arg His Glu Asn
   435 440 445
   Tyr Asp Glu Ala Leu Arg Leu Leu Arg Lys Ala Thr Ala Leu Pro Ala
   450 455 460
   Arg Arg Ala Glu Tyr Phe Asp Gly Ser Glu Pro Val Gln Asn Arg Val
   465 470 475 480
   Tyr Lys Ser Leu Lys Val Trp Ser Met Leu Ala Asp Leu Glu Glu Ser
   485 490 495
   Leu Gly Thr Phe Gln Ser Thr Lys Ala Val Tyr Asp Arg Ile Leu Asp
   500 505 510
   Leu Arg Ile Ala Thr Pro Gln Ile Val Ile Asn Tyr Ala Met Phe Leu
   515 520 525
   Glu Glu His Lys Tyr Phe Glu Glu Ser Phe Lys Ala Tyr Glu Arg Gly
   530 535 540
   Ile Ser Leu Phe Lys Trp Pro Asn Val Ser Asp Ile Trp Ser Thr Tyr
   545 550 555 560
   Leu Thr Lys Phe Ile Ala Arg Tyr Gly Gly Arg Lys Leu Glu Arg Ala
   565 570 575
   Arg Asp Leu Phe Glu Gln Ala Leu Asp Gly Cys Pro Pro Lys Tyr Ala
   580 585 590
   Lys Thr Leu Tyr Leu Leu Tyr Ala Gln Leu Glu Glu Glu Trp Gly Leu
   595 600 605
   Ala Arg His Ala Met Ala Val Tyr Glu Arg Ala Thr Arg Ala Val Glu
   610 615 620
   Pro Ala Gln Gln Tyr Asp Met Phe Asn Ile Tyr Ile Lys Arg Ala Ala
   625 630 635 640
   Glu Ile Tyr Gly Val Thr His Thr Arg Gly Ile Tyr Gln Lys Ala Ile
   645 650 655
   Glu Val Leu Ser Asp Glu His Ala Arg Glu Met Cys Leu Arg Phe Ala
   660 665 670
   Asp Met Glu Cys Lys Leu Gly Glu Ile Asp Arg Ala Arg Ala Ile Tyr
   675 680 685
   Ser Phe Cys Ser Gln Ile Cys Asp Pro Arg Thr Thr Gly Ala Phe Trp
   690 695 700
   Gln Thr Trp Lys Asp Phe Glu Val Arg His Gly Asn Glu Asp Thr Ile
   705 710 715 720
   Lys Glu Met Leu Arg Ile Arg Arg Ser Val Gln Ala Thr Tyr Asn Thr
   725 730 735
   Gln Val Asn Phe Met Ala Ser Gln Met Leu Lys Val Ser Gly Ser Ala
   740 745 750
   Thr Gly Thr Val Ser Asp Leu Ala Pro Gly Gln Ser Gly Met Asp Asp
   755 760 765
   Met Lys Leu Leu Glu Gln Arg Ala Glu Gln Leu Ala Ala Glu Ala Glu
   770 775 780
   Arg Asp Gln Pro Leu Arg Ala Gln Ser Lys Ile Leu Phe Val Arg Ser
   785 790 795 800
   Asp Ala Ser Arg Glu Glu Leu Ala Glu Leu Ala Gln Gln Val Asn Pro
   805 810 815
   Glu Glu Ile Gln Leu Gly Glu Asp Glu Asp Glu Asp Glu Met Asp Leu
   820 825 830
   Glu Pro Asn Glu Val Arg Leu Glu Gln Gln Ser Val Pro Ala Ala Val
   835 840 845
   Phe Gly Ser Leu Lys Glu Asp
   850 855

## Description

### Technical Field

This invention relates to a transcription control factor and the like, and more particularly, to a factor or a peptide capable of controlling the transcription ascribed to nuclear hormone receptors.

### Background Art

Hormones, lipid soluble vitamins and the like play an important role in homoestatic maintenance, energy metabolism, differentiation, growth and the like of organisms. Receptors such as of these hormones and the like are transcription control factors existing in nuclei and bind with particular sites of chromatin DNA to control the transcription reaction of genes. In most cases, where ligands such as hormones and the like do not bind with receptors, transcription is repressed from occurring. Once a ligand, such as a hormone, binds to a receptor, the transcription is activated through a change of a chromatin structure. It has been reported that many factors called co-activators and co-repressors work to form complexes on the way to a transcriptor from a nuclear receptor. An unliganded receptor binds to a co-repressor containing a histone deacetylase, thereby repressing gene expression. On the other hand, when the receptor structure is changed through binding with a ligand, the co-repressor complex is released and, instead, a co-activator complex containing a histone acetylase is recruited. Mention is made, as an instance of such a co-activator, of Skip (i.e. Ski interacting protein, and also called N-CoA62), which is directly bound to several types of nuclear receptors (e.g. vitamin 3 receptor, retinoic acid receptor, estrogene receptor and glucocorticoid receptor) to strengthen the gene expression transmitted through these nuclear receptors (Baudino, T.A., Kraichely, D.M., Jefcoat, S.C., Jr., Winchester, S. K., Partridge, N.C., and MacDonaldo, P.N. (1998) J. Biol Chem 273(26), 16434-41, MacDonald, P.N., Baudio,T.A., Tokumaru, H., Dowd, D.R., and Zhang, C. (2001) Steroids 66(3-5), 171-6).

### Disclosure of Invention

As stated hereinabove, although the outline of the transcription control mechanism through nuclear receptors is now being clarified, it has been left yet to elucidate factors taking part in the mechanism. Therefore, the invention has for its object the provision of a transcription control factor, and particularly, a novel factor that is able to take part in the transcription control of nuclear transcription receptors.

The present inventors have made studies on cloning of a human homolog of Drosophila crn (crooked neck) gene and also on functional analyses thereof, through which it was found that the human homolog takes part in transcription control through the nuclear receptor. It will be noted that the Drosophila crn gene itself is a gene that reaches a maximum level of expression at an initial embryonic stage. It has been reported that the inactivation of this gene causes defects of embryogenesis and mainly influences the development of the nervous system (Zhang, K., Smouse, D., and Perrimon, N. (1991) Genes Dev 5(6), 1080-91). One specific characteristic of crn protein is that 16 copies of a longitudinally directed tetratricopeptide repeat (TRP) exist. TRP has been found in versatile proteins and is a synonymous 34 amino acid repeat motif that has been spread in the course of evolution. The processes in which TPR protein takes part include cell cycle control, transcription repression, stress response, protein kinase repression, and protein transport (Lamb, J. R., Tugendreich, S., and Hieter, P. (1995) Trends Biochem Sci 20(7), 257-9).

Like the above-indicated Drosophila crn gene, a number of TPR's exist in the human homolog of the crn gene although differing in the number of copies that is at 15. The human crn gene which has been cloned by us is in coincidence with a gene (Yoshimichi et al., Journal. Biol. Chem. 275:34931-34937) coded with a protein (XAB2), which takes part in the repair of transcription, conjugation DNA related to the hitherto reported transcription. Nevertheless, we have newly discovered that the product from this gene takes part in transcription repression. Especially, because the gene product functions as a repressor after bonding with HDAC (histone deacetylase), this is called herein "HDART (a HDAC associated repressor TRP) protein. Moreover, we have found that HDART directly binds to Skip serving as a transcription coactivator of the afore-indicated nuclear receptor to repress the transcription of the nuclear receptor. In addition, it has been also clarified that HDART is one of transcription corepressors of the nuclear receptor, and binds with HDAC so that it is able to strongly suppress the transcription through the histone deacetylation of HDAC. On the other hand, it has also been confirmed that a dominant negative peptide is obtained and this peptide serves to activate the transcription, unlike full-length HDART. More particularly, the invention is based on newly discovered, various functions of HDART and is directed to those particularly recited below.
(1) The description is directed to DNA coded with a transcription repressing factor, including (A) DNA coded with a protein having an amino acid sequence indicated at Sequence No. 2 or (B) DNA consisting of a base sequence indicated at Sequence No. 1.
(2)The description is directed to DNA coded with a transcription Repressing factor, including (A) DNA coded with a protein having such an amino acid sequence that one or plural amino acids are substituted with, deleted from, inserted into and/or added to the amino acid sequence indicated in Sequence No. 2 or (B) DNA hybridized with DNA consisting of the base sequence indicated at Sequence No. 1 under stringent conditions.
(3) The description is directed to a transcription inhibitor coded with DNA recited in (1) or (2) above.
(4) The description is directed to a transcription repressing factor recited in (3) above and capable of repressing the transcription ascribed to nuclear hormone receptors.
(5) The invention is directed to DNA coded with a peptide capable of activating transcription, including
   (A) DNA coded with a peptide consisting of the amino acid sequence from position 1 to 179 in the Sequence No. 2 or
   (B) DNA consisting of a base sequence including from 1 to 537 bases in the Sequence No. 1.
(6) The invention is directed to DNA coded with a peptide capable of activating transcription, including (A) DNA coded with a peptide having such an amino acid sequence that one or plural amino acids are substituted with, deleted from, inserted into and/or added to the amino acid sequence from position 1 to 179 in the Sequence No. 2, or (B) DNA hybridized with DNA consisting of the base sequence from position 1 to 537 in the Sequence No. 1.
(7) The invention is directed to a transcription activated peptide coded with DNA recited in (5) or (6) above.
(8) The description is directed to DNA having at least 15 nucleotide length selected among DNAs recited in any one of (1), (2), (5) or (6) above.
(9) The invention is directed to a vector inserted with the DNA recited in any one of (5) or (6).
(10) The invention is directed to a host cell holding DNA recited in any one of (5) or (6) above or the vector recited in (9) above.
(11) The description is directed to an antibody capable of binding the factor recited in (3) above or the peptide recited in (7) above.
(12) The description is directed to an oligonucleotide probe that is hybridized with the DNA recited in any one of (1), (2), (5) or (6) and having at least 10 nucleotide length.
(13) The description is directed to a substrate fixed with any of the following (A) to (D).
   (A) The oligonucleotide probe recited in (12) above.
   (B) The transcription repressing factor recited in (3) or (4) above or the partial peptide thereof.
   (C) The transcription activated peptide recited in (7) above, or the partial peptide thereof.
   (D) The antibody recited in (11) above.

The embodiments of the invention are described in more detail. The abbreviations used in this specification are illustrated beforehand: HAT (histone acetyltranspherase or histone acetylase); HDAC (histone deacetylase or histone deacetylizing enzyme); DAPI (4 ,6 -diamidino2-phenylindole); RAR (retinoic acid receptor); GR (glucocorticoid receptor); DBD (DNA-binding domain); AD (activated domain); and ATRA (all-trans retinoic acid).

The description relates to a factor concerning transcription control. This transcription control factor includes a transcription repressing factor and an activating factor. First, the transcription repressing factor is described. An instance of the transcription repressing factor of the invention includes HDART whose amino acid sequence is one indicated in Sequence No. 2. It should be noted, however, that the transcription repressing factor according to the invention is not limited to the HDART indicated in Sequence No. 2, but encompasses, within a range as exhibiting transcription repressing activity, proteins having such an amino acid sequence that one or plural amino acids are substituted with, deleted from, inserted into and/or added to the amino acid sequence indicated in Sequence No. 2, and also proteins coded with DNA which is hybridized with DNA (Sequence No. 1) coded with HDART under stringent conditions.

The HDART protein is expressed within nuclei of human cells and can be obtained from the nuclei of human cells. Starting human cells are not critical, and mention is made, for example, of 293 cells, for which it is apparent to endogenously express HDART.

The preparation of proteins similar to HDART, which have amino acid substituents, can be carried out by use, for example, of known techniques such as a phage library screening technique (Molecular Cloning 3rd Ed, Chapter 2, pp. 2.1-2.117), a polymerase chain reaction (PCR: Molecular Cloning 3rd Ed, Chapter 8, pp.8.1-8.126) technique. More particularly, such a protein is obtained by using DNA (Sequence No. 1) coded with HDART or part thereof as a probe or primer to obtain DNA provided with Sequence No. 1 and a homology, followed by producing a protein based on the DNA. The thus obtained protein usually has high homology with respect to HDART and the amino acid sequence. This high homology means the coincidence of the base sequence at at least 40% or over, preferably, 60% or over, more preferably 80% or over, further more preferably 90% or over, still further preferably 95% or over and most preferably at least 97% or over (e.g. from 98 to 99%).

The "stringent hybridization conditions" are ones that can be appropriately selected to those skilled in the art. For instance, mention is made of hybridization which is carried out by performing pre-hybridization in a hybridization solution containing 25% formamide, or 50% formamide under severer conditions, 4 × SSC, 50mM of Hepes with a pH of 7.0, a 10 × Denhalt's solution, 20 µ µ g/ml of modified sermon sperm at 42°C overnight, adding a labeled probe to the solution, and allowing to stand at 42°C overnight. The wash fluid and temperature conditions for subsequent washing include 1 × SSCm 0.1% of SDS and about 37°C, and severer conditions include 0.5 × SSC, 0.1% of SDS and about 42°C with further severer conditions including 0.2 × SSC, 0.1% of SDS and about 65°C. The combinations of SSC, SDS and temperature are mentioned for illustration only, and may be appropriately changed to those skilled in the art.

The homology of sequence can be determined by utilizing the program (Altschul et al. J.Mol. Biol. 215:403-420, 1990) of BLASTn (nucleic acid level) or BLASTx (amino acid level). The program is based on the algorithm BLAST of Karlin and Altschul (Proc. Natl. Acad. Sei. USA 87:2264-2268, 1990, Proc. Natl. Acad. Sei. USA 90:5873-5877, 1993). Where a base sequence is analyzed based on BLASTN, parameters are, for example, such that score =100 and wordlength = 12. On the other hand, where an amino acid sequence is analyzed based on BLASTX, parameters are, for example, such that score = 50 and wordlength = 3. Where an amino acid sequence is analyzed using Gapped BLAST programs, the analysis can be carried out in a manner as described by Altschul et al. (Nucleic. Acids. Res. 25:3389-3402, 1997). When using BLAST and Gapped BLAST programs, default parameters of the respective programs are used. The specific procedures of these analyses are known in the art.

### (http://www.ncbi.nlm.nih.gov.)

In case where the sequence of Sequence No. 2 is artificially modified, it is considered that the modification is generally within 10% of the total amino acid, preferably within 5% of the total amino acid and more preferably within 1% of the total amino acid. Within a range where the transcription repression activity can be kept, the amino acid sequence may be substituted to such an extent exceeding the above-indicated percentage of modification. This technique of artificially modifying the amino acid sequence can be executed, for example, by known procedures including a deletion-mutant preparation procedure, a PCR procedure, site-directed mutagenesis and the like. It will be noted that whether or not a protein modified in this way has transcription repression activity like HDART can be determined by use of many reporter analyzing methods described in examples appearing hereinafter to analyze the transcription repressing ability.

The HDART and a protein similar thereto have such transcription repression activity as set out hereinabove, so that this function is utilized to repress the transcription of a desired gene through combination with a desired transcriptor. The transcriptor may be either an in vitro transcription system or an vivo transcription system. Since the transcription repressability of HDART is autonomous, the transcription inhibitor of the invention is able to repress transcription when used singly. In this connection, however, HDART has no bindability on DNA and should preferably be used as a fusion protein fused with a DNA-binding region. HDART has bindability on HDAC and thus, is able to recruit HDAC thereby intensely repressing transcription through histone deacetylase activity. In this sense, the transcription repressing factor of the invention can inhibit transcription by using along with HDAC or by deriving HDAC. It is known that HDAC has type I (HDAC1, HDAC2, HDAC3) and type II (HDAC4, HDAC5, HDAC6, HDAC7, HDAC8). The HDART of the invention is considered to bind to HDAC2, HDAC5, HDAC7, HDAC8 and the like. Thus, in the practice of the invention, HDAC2, HDAC5, HDAC7, and HDAC8 can be conveniently used. However, limitation should not be placed on the use of these HDACs alone, but HDACs belonging to either of type I or II may also be usable.

Since HDART is able to repress nuclear receptor transcription, a transcriptor working through a nuclear receptor can be conveniently mentioned for a transcriptor capable of being repressed by means of the transcription repressing factor of the invention. As the nuclear receptor, mention is favorably made of retinoic acid receptor and glucocorticoid receptor. Moreover, nuclear receptors against hormones and fat-soluble vitamins, such as retinoin X receptor, vitamin D receptor, androgen receptor, esterogen receptor, tiroid hormone receptor and the like, may also be included within the range where the factor of the invention ensures repression the transcription. Hence, the transcription repressing factor of the invention is useful for treating diseases caused by the incordination of transcription from such nuclear receptors, particularly, by the excess promotion of the transcription. Especially, as is shown in examples appearing hereinafter, because it has been made clear that HDART represses the transcription of retinoic acid receptor and also inhibits the differentiation elicited from the transcription of the retinoic acid receptor, the transcription repressing factor of the invention may be applied for a curative medicine against diseases that are ascribed to increased differentiation based on the transcription of the retinoic acid receptor.

The invention relates to DNA coded with the transcription repressing factor. For the DNA, mention is made of DNA consisting of the base sequence indicated, for example, in Sequence No. 1 although not limitative thereto. Moreover, there may also be used DNA coded with the amino acid sequence indicated in the Sequence No. 2, DNA coded with a protein having an amino acid sequence wherein one or plural amino acids are substituted with, deleted from, inserted into and/or added to the amino acid sequence indicated in Sequence No. 2, and DNA hybridized with DNA consisting of the base sequence indicated in Sequence No. 1 under stringent conditions.

The above DNA is obtained by using DNA indicated Sequence No. 1 or part thereof as a probe or primer and subjecting to hybridization or polymerase chain reaction (PCR), for example, with cDNA library of human cells (e.g. island cells of the human pancreas as shown in Example 1 appearing hereinafter are mentioned). Alternatively, the DNA may be obtained from RT-PCR (Molecular Cloning 3rd Ed, Chapter 8, Protocol 8, pp. 8.46-8.53) by another method wherein mRNA of the human cells is used as a template and part of DNA indicated in Sequence No. 1 is provided as a primer. It will be noted that although the instance of the human cells has been illustrated above, the preparation may be carried out using, aside therefrom, other mammal cells, the eucaryotic cells and the like. In addition, the hybridization conditions for isolating DNA are those indicated hereinbefore.

Aside from the above-stated procedure of cloning the DNA, the DNA may be formed by synthesizing DNA indicated in Sequence No. 1 and complementary strands by use of a DNA synthesizer, respectively, followed by annealing.

The above DNA is coded with the transcription repressing factor and can thus be used as a tool for producing the transcription repressing factor or a tool for expressing the transcription repressing factor after introduction into the cells or individuals. When using for this purpose, it is preferred to incorporate the DNA into an expression vector or the like. The type of expression vector can be appropriately selected depending on the translation system used in the production of protein or the cell to be introduced.

For the production of the transcription repressing factor used the DNA-incorporated vector, the vector is first introduced into host cells, followed by cultivating the host cells. In this way, the transcription repressing factor is produced in the host cells. The procedure of introducing the vector into the cells may be appropriately selected depending on the type of cell used. For instance, there may be used a virus vector or phage-mediated biological technique, chemical techniques such as a calcium phosphate method, a lipofection method and the like, physical techniques such as a gene gun method and an electropolation method, and the like. The protein produced in the host cell can be used after purified (e.g. by affinity purification), if necessary.

The DNA-incorporated expression vector may also be used for the purpose of repressing a desired transcription within the cells or within an individual. More particularly, when the expression vector is introduced into the cells or an individual to express the transcription repressing factor, a desire transcription system can be repressed. Especially, HDART has autonomous transcription repression ability, so that the transcription repressing factor of the invention shows the transcription repressing activity when used singly. It will be noted that HDART has no bindability on DNA, and it is preferred that the transcription repressing factor of the invention is expressed as a fusion protein with a DNA binding region capable of binding on DNA of a control region of a desired gene. In doing so, the transcription of a target gene can be repressed. The transcription repressing factor of the invention has bindability on HDAC, so that when the transcription repressing factor is expressed, HDAC is recruited, thereby repressing the transcription more intensely.

Because the transcription repressing factor coded with DNA according to the invention has the capability of effectively repressing the transcription of nuclear receptors, the vector incorporated with this DNA may be applied to as a composition for treating diseases ascribed to the increased transcription of these nuclear receptors. For the vector intended for the treatment, mention is made, for example, of virus vectors such as retrovirus vector, adenovirus vector, adeno-associated virus vector, vaccinia virus vector, lenti virus vector, herpes virus vector, alpha virus vector, EB virus vector, papiroma virus vector, foamy virus vector and the like.

The invention relates to a peptide, selected among the transcription repressing factors, which is capable of activating transcription unlike in the above case. HDART functions, in a full length thereof, as a transcription repressing factor. On the contrary, a dominant negative peptide of HDART functions as a transcription activator. Examples of the dominant negative peptide include a peptide coded with four TPR's (hereinafter abbreviated as "N4TPR") at the N terminus, i.e. a peptide consisting of an amino acid sequence from position 1 to position 179 in Sequence No. 2, although not limitative thereto. So far as transcription activity is possessed, those peptides wherein one or plural amino acids are substituted with, deleted from, inserted into and/or added to the amino acid sequence from position 1 to position 179 in Sequence No. 2 may also be included.

The dominant negative peptide can be prepared by synthesizing a peptide based on DNA consisting of a base sequence from 1 to 537 of Sequence No. 1. Moreover, this peptide and a peptide having a substituent in the base subsequent from 1 to 537 can be prepared by synthesizing a peptide based on DNA wherein codon is changed by adding a variation (substitution, deletion, insertion and/or addition) to the base sequence from 1 to 537 in the amino acid sequence.

The above peptide has transcription activation activity and can be used to promote the transcription of desired transcription systems. Especially, as shown in examples, because N4TPR of HDART activates the transcription of nuclear receptors, it may be used to promote the transcription of these nuclear receptors. For the nuclear receptor, mention is favorably made of nuclear receptors on which Skip acts, e.g. retinoic acid receptor, glucocorticoid receptor, vitamin D receptor, esterogen receptor, and the like. Within a range where the peptide of the invention ensures transcription activation, nuclear receptors against hormones or fat-soluble vitamins, such as retinoin X receptor, androgen receptor, tiroid hormone receptor and the like, may also be included.

It is shown in examples appearing hereinafter that the transcription activation activity of retinoic acid receptor by the action of the N4TPR is higher than the transcription activation activity caused by ATRA. Hence, a differentiation-inducing therapy of malignancies such as leukemia is now carried out through the transcription activation activity of retinoic acid receptor promoted by means of ATRA. Moreover, vitamin A, and derivatives thereof including ATRA have started to be in use for curing, aside from leukemia, hepatic cell carcinoma (Okuno, M. et al., (2002) Front Biosci 7, 204-18), ovarian cancer (Zhang D. et. al., (2000) J, Cell Physiol 185(1), 1-20), thyroid cancer (Schmutzler C. and Kohrle J. (2000) Tyroid 10(5), 393-406), skin cancer (Niles R.M. (2000) Nutrition 16(11-12), 1084-9), pancreatic cancer (Riecken E.O. and Rosewicz S. (1999) 10 Suppl 4, 197-200) and the like. In place of this ATRA or in combination with ATRA, the peptide of the invention may be used.

The invention also relates to DNA coded with a peptide having such transcription activation activity as stated hereinabove. Mention is made, as this DNA, of DNA coded with a peptide consisting of an amino acid sequence from 1 to 179 of Sequence No 2, for which an instance includes DNA consisting of a base sequence made of bases of Sequence Nos. 1 to 537. Although not limitative to this, there may also be included, so far as they have transcription activation activity, DNA coded with a peptide having an amino acid sequence wherein one or plural amino acids are substituted with, deleted from, inserted into and/or added to an amino acid sequence from 1 to 179 of Sequence No. 2, DNA consisting of a base sequence including from 1 to 537 bases in Sequence No. 1 and DNA hybridized under stringent conditions.

The DNA coded with the transcription activated peptide can be prepared by obtaining DNA coded with the afore-indicated transcription inhibitor and causing, for example, a C terminus side to be deleted. Alternatively, the DNA may be prepared by synthesis through a DNA synthesizer.

The DNA can be used for the purpose of producing the transcription activated peptide or expressing the transcription activated peptide after introduction into cells or individuals. Where the DNA is used so as to produce a peptide, it is preferred to incorporate in an expression vector. In this case, the expression vector can be appropriately selected depending on the transcription/translation system for producing a peptide. This transcription/translation system may be either in vitro or in vivo. With the in vivo system, the expression vector incorporated with the DNA is introduced into cells and the cells are cultivated to produce the transcription activated peptide within the cell. The procedure of introducing into cells are similar to those stated hereinbefore.

Where the DNA is used for the purpose of expressing the transcription activated peptide after introduction into cells or individuals, the DNA may be directly introduced into cells or the like for transient expression or stable expression after insertion into chromosomes, or may be introduced into cells or the like after incorporation into the expression vector.

As stated hereinabove, the transcription activated peptide may be applicable to a differentiation-inducing therapy of malignancy like ATRA, and thus can be utilized for the therapy wherein instead of directly using the transcription activated peptide, this DNA is injected into or applied to a patient to express the transcription activated peptide. To this end, it is preferred to use the DNA after incorporated into a vector for carrying the DNA to a desired tissue or cells. For the vector used for this type of therapy, a virus vector such as the afore-indicated retrovirus vector or the like may be used.

As stated hereinabove, the DNA coded with the transcription repressing factor of the invention is able to be modified into DNA coded with the transcription activated peptide by shortage of the DNA coded at the N terminus side thereof. Besides, the DNA coded with the transcription repressing factor or the DNA coded with a transcription activated peptide can be provided as a more shortened fragment for utilization as a probe for hybridization, a PCR primer or a ribozyme derivative. Where part of the DNA is used for this purpose, it is preferred that the fragment has a length capable retaining specificity enough to use as a probe or the like, particularly, a 15 nucleotide length. The invention provides an olegonucleotide probe having at least 10 nucleotide length by hybridization with the DNA of the invention (e.g. DNA or the like indicated at Sequence No. 1). For instance, such a polynucleotide includes one DNA made of a base sequence indicated in Sequence No. 1 or one specifically hybridized with a complementary strand thereof. The term "specifically hybridized" used herein means that in hybridization, no significant cross hybridization with DNA coding other type of protein therewith takes place. The above-mentioned probe and primer can be used for cloning or the like of DNA coded with a transcription repressing factor or the like.

The description relates to an antibody capable of binding on the transcription repressing factor or transcription activated peptide. The antibody of the invention may be either a polyclonal antibody or a monoclonal antibody provided that it is specifically bindable on the transcription repressing factor or transcription activated peptide. The polyclonal antibody can be prepared by mixing the protein of the invention or a partial peptide with Freund's adjuvant, if necessary, immunizing a nonhuman animal such as rabbit, goat, guinea pig or the like according to a known procedure, and collecting serum from the peripheral blood of the immunized animal after confirming that the antibody level increases. On the other hand, the monoclonal antibody is prepared by immunizing an animal such as mouse according to a known procedure using the transcription inhibitor, transcription activated peptide or a partial peptide thereof, removing the spleen or the lymph nodes from the immunized animal whose antibody level has increased, and creating a fusion of the antibody-producing cells and the mieroma cells in the tissue to prepare a hybridoma. Thereafter, an antibody produced from the hybridoma is collected from a culture supernatant liquid to obtain the monoclonal antibody.

These antibodies can be utilized not only for affinity purification of the transcription repressing factor or transcription activated peptide, but also for immunological analysis of an expressed amount of the transcription repressing factor in various cells or for inhibiting the transcription repressing factor.

The description provides a substrate fixed with the oligonucleotide thereon. The substrate is provided as a biochip, enabling one to analyzing the expressing condition of DNA of the invention in a test organism (cells).

In a preferred embodiment, DNA of the invention or description (e.g. DNA indicated in Sequence No. 1 or a partial DNA region thereof) is amplified from a test organism (cells). Next, a substrate fixed with a nucleotide probe to be hybridized with the DNA is provided. Thereafter, the DNA and the substrate are brought into contact with each other. When the DNA hybridized with the nucleotide probe fixed on the substrate is detected, the expressed condition of the DNA of the invention can be analyzed.

Such a method includes, for example, a DNA array technique. The preparation of a DNA sample from a test organism (cells) is feasible by a procedure known to the skill in the art. In a preferred embodiment of preparing the DNA sample, the preparation is possible on the basis of the chromosome DNA extracted from the cells. For the preparation of the DNA sample of this procedure from the chromosome DNA, it is possible to prepare the DNA of the invention by PCR or the like using the chromosome DNA as a template using, for example, a primer to be hybridized with the DNA of the invention. The thus prepared DNA sample may be labeled for detection by a method known in the art, if necessary.

The term "substrate" used in this description means a plate-shaped material capable of fixing nucleotide thereon and is ordinarily called chip. In the practice of the description, the nucleotide includes oligonucleotide and polynucleotide. Although the substrate of the description is not critical so far as it is able to fix the nucleotide thereon, a substrate ordinarily used in the DNA array technique is favorably used.

In general, the DNA array is constituted of several thousands of nucleotides printed on a substrate at high density. Usually, these DNA's are printed on a surface layer of a non-porous substrate. The surface layer of the substrate is usually made of glass, and a porous membrane such as, for example, a nitrocellulose membrane may be used.

In the practice of the description, an array based on the oligonucleotide and developed by Affymetrix Inc., is exemplified as the fixing (array) method of nucleotide. In the array of the oligonucleotide, the oligonucleotide is synthesized in situ. For instance, the in-situ synthetic methods of oligonucleotides have been already known including, for example, a photolithographic technique (Affymetrix Inc.), an ink-jet technique (Rosetta Inpharmatics LLC Co.) of fixing chemical substances, and the like. All of these techniques can be utilized for the fabrication of the substrate of the invention.

The type of nucleotide probe fixing on the substrate is not critical provided that the DNA of the invention is able to be detected therewith. More particularly, the probe is, for example, one which is specifically hybridized with the DNA indicated at Sequence No. 1. If specific hybridization is possible, the nucleotide probe should not be fully complementarily relative to the DNA of the invention.

Where the nucleotide is fixed in the practice of the description, the length of the nucleotide bound on the substrate is generally 10 to 100 bases, preferably 10 to 50 bases, and more preferably 15 to 25 bases.

In the practice of the description, the DNA sample and the substrate are then brought into contact with each other. According to this procedure, the DNA sample is hybridized with the nucleotide probe. The reaction solution and reaction conditions for the hybridization may change depending on the factors including the length of nucleotide to be fixed on the substrate and the like, and the hybridization can be generally conducted by the procedure well known to the skill in the art.

Next, according to the description, the presence or absence, or the intensity of the hybridization between the DNA sample and the nucleotide probe fixed on the substrate is detected. This detection can be carried out by reading, for example, a fluorescent signal such as by a scanner or the like. It will be noted that in the DNA array, it is usual to refer DNA fixed on a slide glass to as probe and DNA labeled in solution to as target. Accordingly, the nucleotide fixed on the substrate is set out as nucleotide probe in this specification. In the above procedure, the thus detected hybridization intensity is compared with a control, if necessary. For the procedure, mention is made, for example, of a DNA array method (Tactics of SNP Gene Variation, by Kenichi Matsubara and Yosiyuki Sakaki, published by Nakayama Shoten, p. 128-135, Nature Genetics (1999)22: 164-167) and the like, which can be appropriately carried out by those skilled in the art with reference to known literature or the like.

The description provides a substrate, on which the protein of the invention or a partial piece of the protein is fixed. When using such a substrate as a biochip, it becomes possible to detect molecules binding the protein of the invention therewith, to screen a HDAS inhibiting compound and the like.

In general, a protein fixed on a substrate is called protein chip. Like DNA chip, the principle is such that a protein is fixed on a slide glass or film in high density for detecting a protein or nucleic acid interacting therewith.

Because of binding with various types of HDAC proteins, the HDART protein of the invention can be applied to screening of a HDAC inhibiting compound. More particularly, the fixing of the HDART protein of the invention on a solid phase surface permits different types of HDAC's to be bound on one face. When different types of compounds are bound on this solid phase surface and HDAC activities are measured, HDAC inhibiting compounds can be screened.

An instance of the HDAC inhibiting compound collected by the screening includes tricostatin A that is used as a medicine in a differentiation-inducing therapy of cancer.

The substrate fixing the antibody of the description can be used as a biochip. The fixing, on a chip surface, of the antibody isolated and purified in high purity enables one to achieve high densification.

In a preferred embodiment of the description, after spotting a sample onto a substrate, proteins or other foreign matters which do not show any affinity for the spotted surface are dissolved out by rinsing. A subsequent detection step is feasible by a skill in the art according to an appropriate, known method while taking the type of substrate and the like, thereby detecting the presence or absence (or a level) of affinity. For one instance, energy absorbing molecules (EAM) are added to the substrate after the ringing, dried and subjected to a mass specrometer (TOF-MS) device to measure molecular weight spectra of a protein bound on the spotted surface.

The fixing of an arbitrary DNA can be conveniently carried out by those skilled in the art according to known procedures.

### Brief Description of Drawings

FIG. 1 shows that HDART is a TPR (Tetra trico peptide repeat) protein, and shows a configuration thereof and the relation with a related gene, also showing a schematic configuration of a primary structure of HDART and TRP, an acidic region, a Skip interacting region and a CRN homology region.
FIG. 2 is a view showing the comparison in CRN homology region between the human HDART and the human CRN.
FIG. 3 is a view showing a HDART/CRN protein-protein polygenic tree. This polygenic tree is built up (development of a software) using a GENETYX-MAC program.
FIGS. 4A to 4B are photographs showing the results identified through immunoprecipitation analyses of the interaction between foreign or internal HDART and foreign Skip.

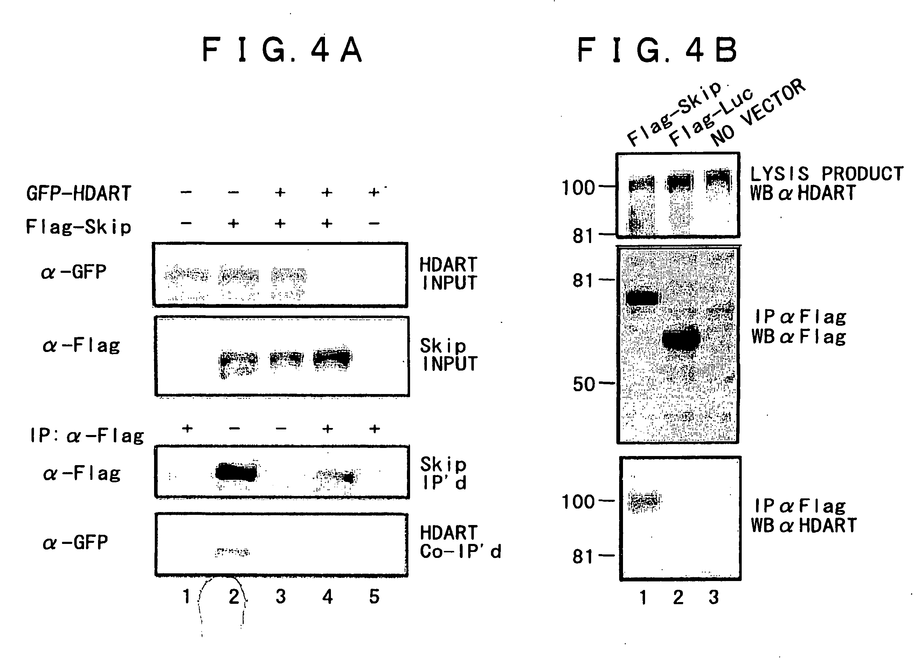

FIG. 4A shows the results of analysis of the interaction between the foreign HDART and foreign Skip. Lane 1 is for GFP-HDART alone, lane 4 is for Flag-Skip alone, and lanes 2, 3 are for both expressed within 293 cells, respectively. Lane 5 is for control cells free of any vector. Lanes 1, 2, 4 and 5 are, respectively, directed to samples immunoprecipitated with an anti-Flag antibody, and lane 3 is directed to a sample immunoprecipitated with control mouse IgG. The upper two panels show expressions of expressed proteins, respectively, and the lower two panels show immunoprcipitates, respectively. It will be noted that the upper panels show the results immunoblotted with an anti-GFP antibody and the lower panels show the results immunoblotted with an anti-Flag antibody.
FIG. 4B shows the results of analyses of the interaction between the internal HDART and the foreign Skip. After introduction of Flag-Skip (lane 1) or Flag-lucipherase (lane 2) into 293 cells internally holding HDART, the cell extract was immunoprecipitated with an anti-Flag antibody. A control text where no vector was introduced was also run simultaneously (lane 3). The expression state of the HDART protein within cells (upper panel), immunoprecipitated Flag (central panel) or the HDART (lower panel) was identified by immunoblotting using an antibody indicated at the left of the panel as "WB".

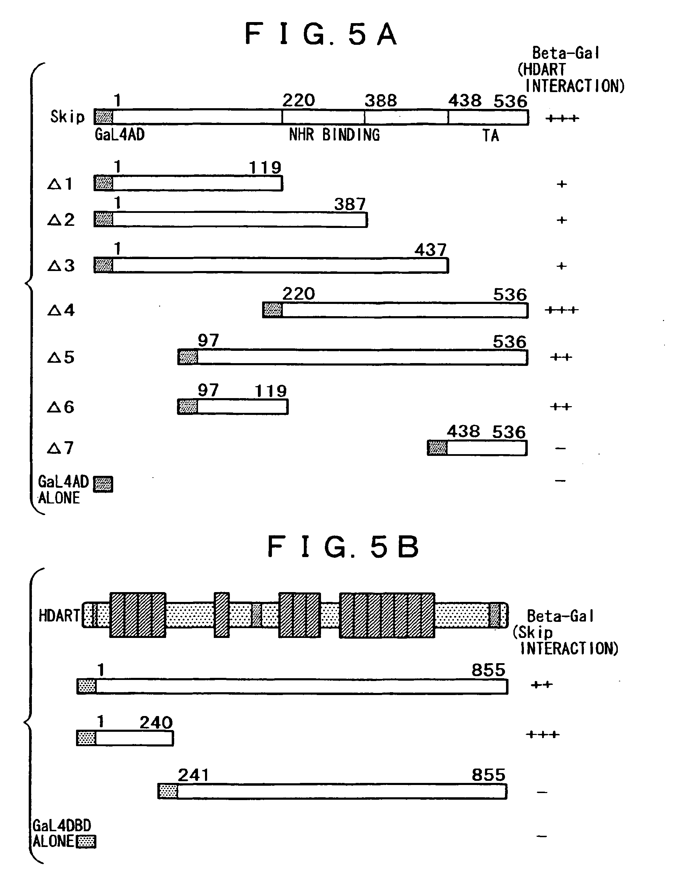

FIGS. 5A to 5B show the results of identification in interaction regions over both Skip and HDART proteins.
FIG. 5A shows mapping of HDART-binding region sites on Skip. The plus (+) sign shows that the interaction is detected based on β-galactosidase activity in a yeast two-hybrid system. The number of the pluses indicates the relative intensity of the interaction. NHR binding: nuclear hormone receptor bound domain, TA: trans activated domain.
FIG. 5B shows the mapping of the Skip-binding region on the HDART. The symbols are the same as defined above.

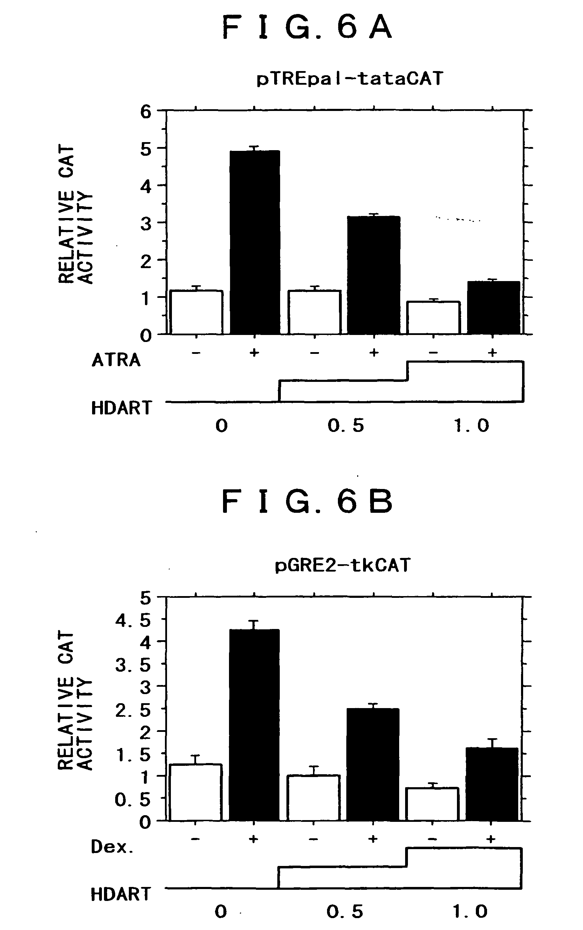

FIGS. 6A to 6B are graphs showing the repression, with HDART, of the transcription activity ascribed to the nuclear hormone (retinoic acid or glucocorticoid).
FIG. 6A shows the results of repression which depends on the concentration of HDART with respect to the transcription activated by means of retinoic acid. A corrected CAT activity is shown a relative to a CAT activity determined on introduction of a vacant vector (1.0 µg) in the absence of a ligand as a reference. An average of three replicated measurements is shown with the error bar indicating S.D.
FIG. 6B shows the results of repression which depends on the concentration of HDART with respect to the transcription undergoing the activation with glucocorticoid.

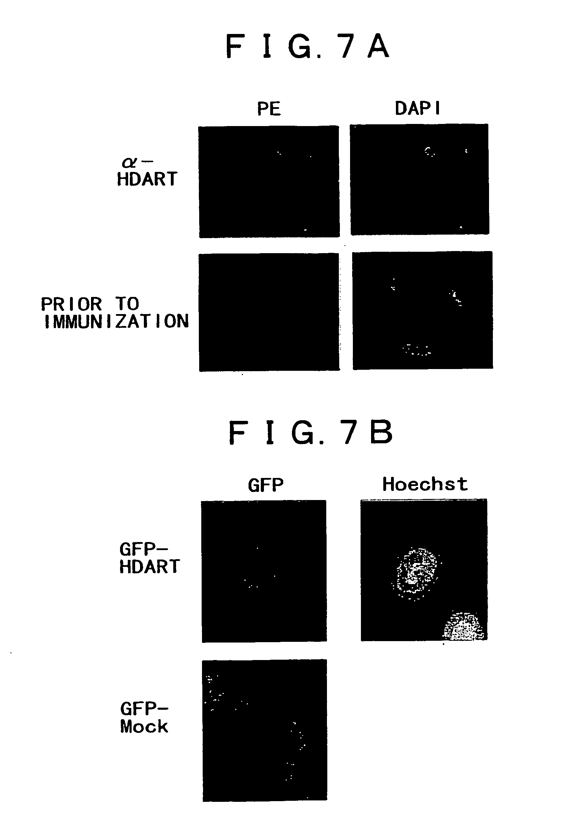

FIG. 7A to 7B are photographs showing the localization within cells of HDART.
FIG. 7A shows the localization of endogenous HDART protein. The left side panels show the results of immunofluorescent staining using an anti-HDART antibody (upper side) or preimmune serum (lower side), and the right side panels show the results of DAPI staining in fields corresponding to the left side panels.
FIG. 7B shows the localization of HDART in viable cells. The results of observation of fluorescence caused by GFP after introduction of a GFP-HDART expression vector (upper left) or a GFP expression vector (lower left) into Hela cells, and the results of visualized the nucleus using the Hoechst 33342 dye (upper right) are shown.

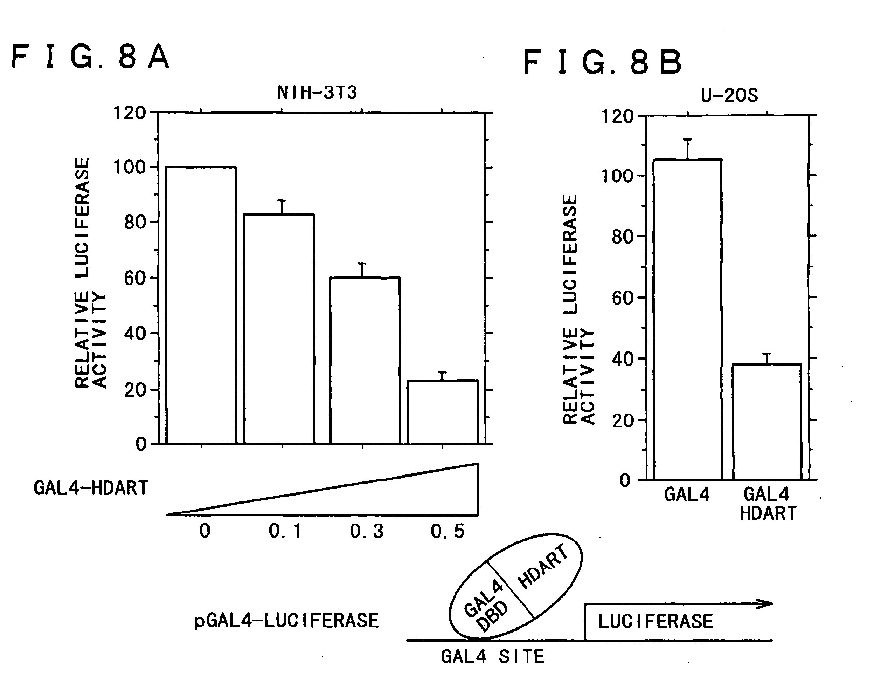

FIGS. 8A to 8B show the autonomous promoter repression activity of HDART.
FIG. 8A shows the results of study of the promoter repression activity when different amounts of Gal4 DBD-HDART expression plasmid (0, 0.1, 0.3, 0.5 µg) are introduced into Ga14 reporter (lucipherase) plasmid-bearing NIH3T3 cells. The corrected lucipherase value relative to the lucipherase activity (100%) obtained on introduction of a vacant vector alone is shown. An average of the results of three replicated experiments is shown, and an error bar indicates S.D.
FIG. 8B shows the results using U-20S cells.

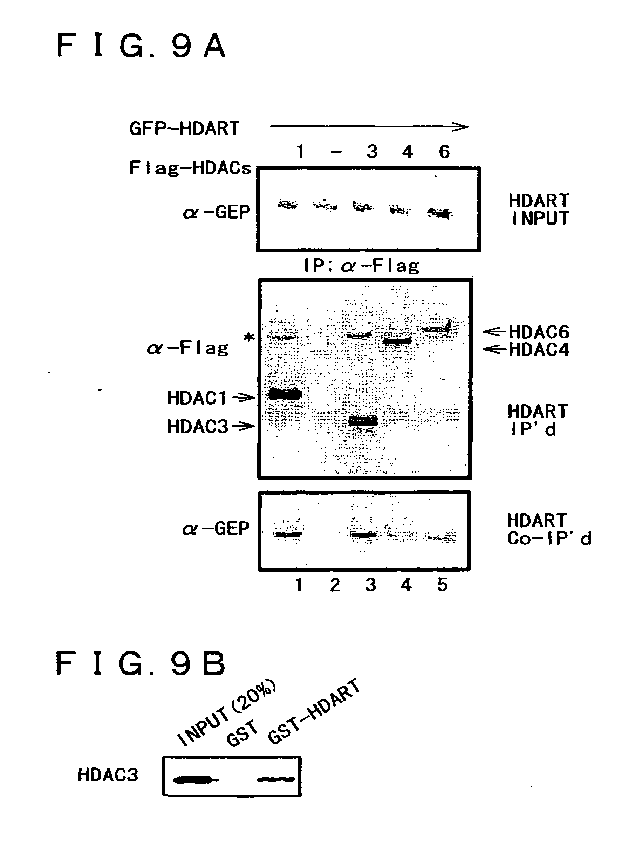

FIGS. 9A to 9B are photographs showing direct interaction between HDART and HDAC.
FIG. 9A shows the interaction between HDART and HDAC. The results of immune blotting with an antibody (anti-Flag antibody or anti-GFP antibody) immunoprecipitated and labeled with an anti-FLAG antibody after co-transfection of a GFP-HDART expression vector and a FLAG-HDACs expression vector ((lane 1; HDAC1 lane 3; HDAC3, lane 4; HDAC4, lane 5; HDAC6) into 293 cells (at the central and lower panels, respectively). It will be noted that lane 2 is for a sample wherein GFP-HDART alone is introduced. The upper panel shows the results of confirmation of the expression of GFP-HDART protein using a sample prior to immunoprecipitation.
FIG. 9B shows the results of the direct interaction of HDART and HDAC3.

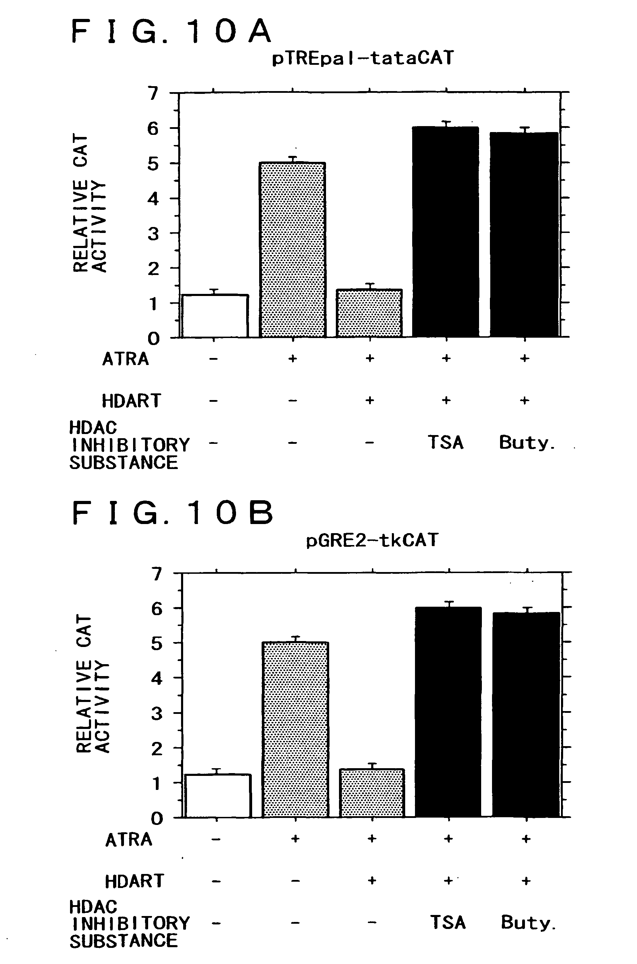

FIGS. 10A to 10B show the inhibition of HDART repressions with two types of HDAC inhibitory substances (tricostatin A, sodium butyrate) (C, D).

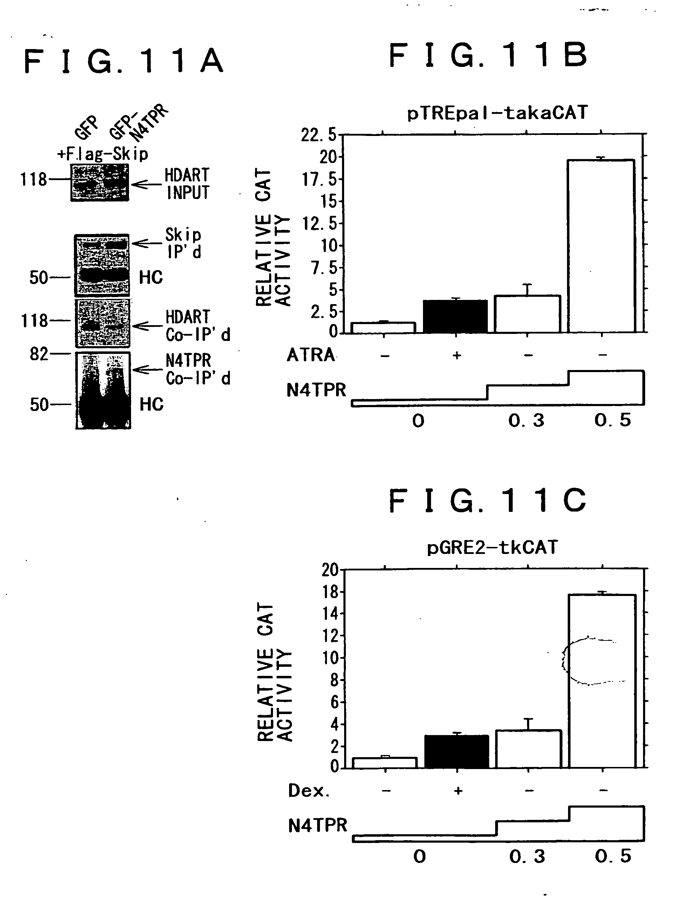

FIGS. 11A to 11C include views and a photograph showing dominant negative effects of four TPR's (N4TPR) at the N terminus of HDART.
FIG. 11A shows the inhibitions of endogenous HDART, Skip and the interaction by the action of N4TPR. The results of a test wherein a cell extract of 293 cells subjected to transfection of Flag-Skip and GFP (lane 1) or GFP-N4TRP (lane 2) was immunoprecipitated with an anti-Flag antibody and the resultant precipitated product was isolated with SDS-Page are shown. The lower three panels, respectively, show immunoprecipitated Skip (upper panel) endogenous HDART (middle panel) and N4TPR (lower panel). The expression of HDART protein prior to the immunoprecipitation is shown at the uppermost panel.
FIG. 11B is a graph showing the activation of the transcription ascribed to the retinoic acid receptor by the action of N4TPR.
FIG. 11C is a graph showing the activation of the transcription ascribed to glucocorticoid receptor by the action of N4TPR.

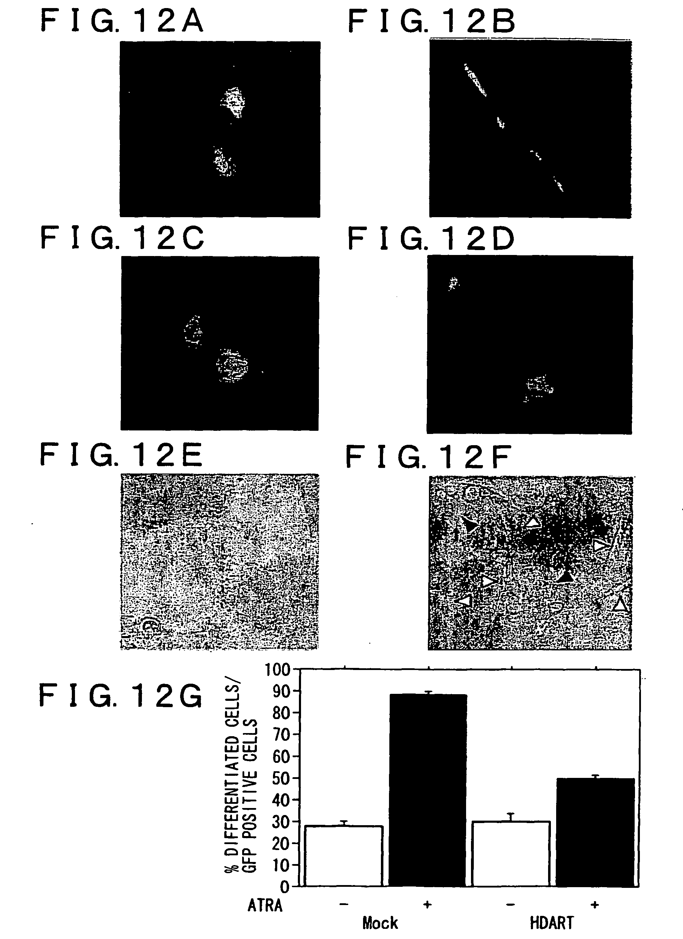

FIGS. 12A to 12G include view and photographs showing the inhibition, with HDART, of differential induction with retinoic acid within MM-1-19-P cells. The MM-1-19-P cells into which a HDART expression vector or a vacant vector had been introduced was analyzed with respect to the differentiation induction in the presence or absence of ATRA (2 µM). In order to identify transduced cells, a GFP vector was co-transfected along with the above-indicated vector. The photographs (12A, 12B, 12C and 12D) of green fluorescences with GFP taken through standard microphotography, and photographs (12E, 12F) taken under a phase-contrast microscope are shown.
FIG. 12A is an ATRA (-) and vacant vector-introduced sample, FIG. 12B is an ATRA (+) and vacant vector-introduced sample, FIG. 12C is an ATRA (-) and HDART expressed vector, FIG. 12D is an ATRA (+) and HDART expressed vector, FIG. 12E is a phase contrast photograph in the same field of view as FIG. 12C, and FIG. 12F is a phase contrast photograph in the same field of view as FIG. 12D. In FIG. 12F, the black arrow indicates a unspecialized GFP positive cell, and a white arrow indicates an specialized GFP positive cell. In FIG. 12G, the graph shows a percentage of morphologically differentiated cells in the GFP positive cells. The results of four independent experiments are submitted as an average and S. D. (error bar) (P<1% between Mock and HDART at ATRA(+), student t-test).

The description is described in detail by way of examples, which should not be construed as limiting the invention thereto.

### [Example 1] Cloning of human HDART

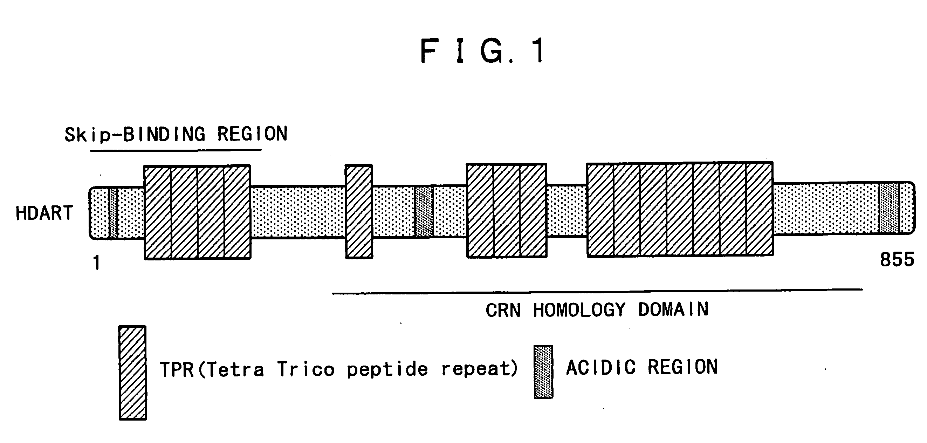

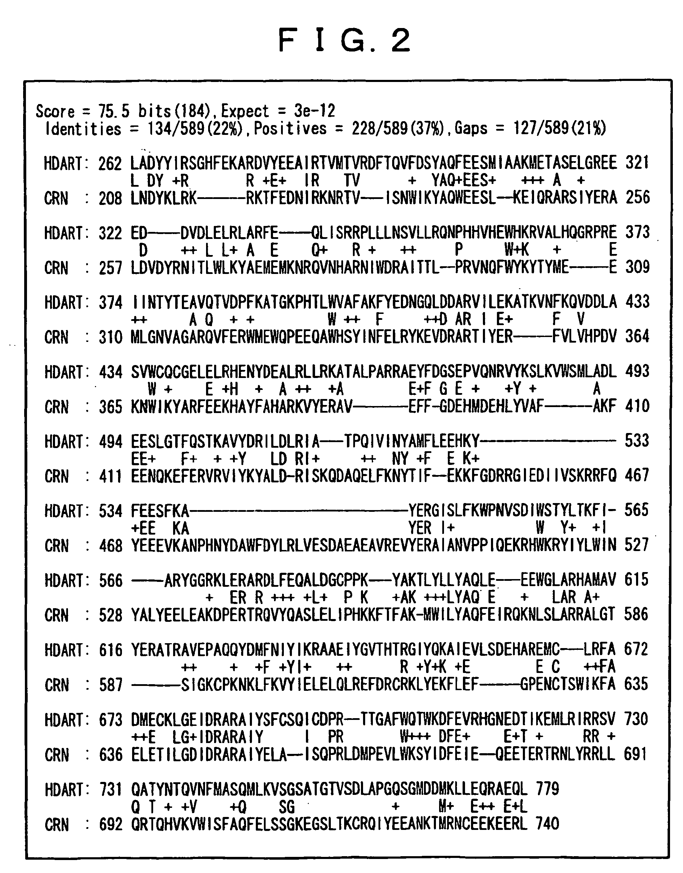

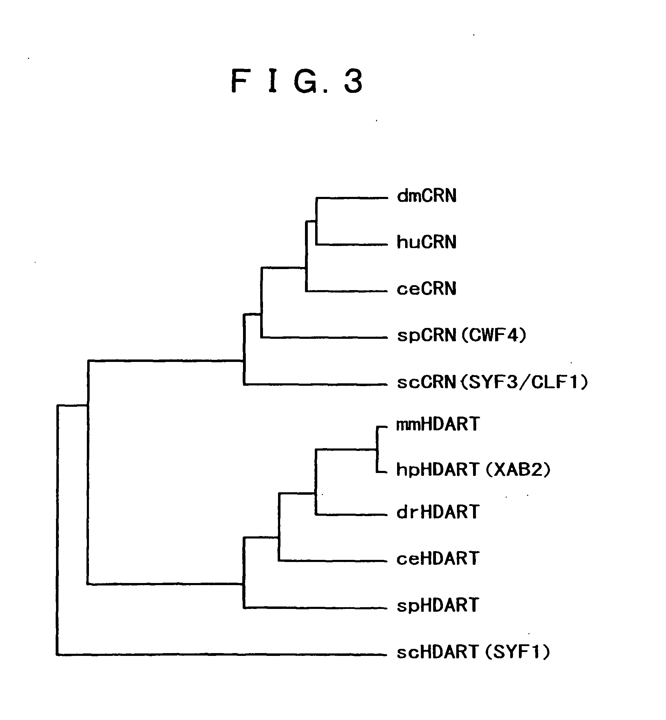

Human homologs of Drosophila crn gene using BLAST data base were checked, revealing that the clone #52930 of human EST (expressed sequence tag) derived from the pancres island has high homology with the crn gene. The perfect sequence of the clone #52930 was determined in a usual manner. Moreover, according to the 5'-RACE procedure (5'-rapid amplification of cDNA ends strategy), this gene was identified as having a full length of cDNA consisting of 2660 bases with one long reading frame. It will be noted that the identified protein functions as a repressor after binding to HDAC (histone deacetylase) as will be described hereinafter and thus is called "HDART (a HDAC associated repressor TPR)" protein. HDART is a highly conserved TPR protein coded with 855 amino acid (FIG. 1). The HDART human protein deduced from the DNA sequence apparently indicates a resemblance to human CRN protein. Especially, the region ranging from 262 residue to 779 residue of the HDART human protein is highly conserved in the HDART and CRN protein (FIGS. 1, 2). The genetic analyses of the proteins over several species revealed that these formed a genetic family (FIG. 3).

### [Example 2] Direct interaction between HDART and transcription coactivator Skip

Attention was paid to several TPR regions existing on the HDART, and it was analyzed if any protein capable of interaction with HDART via these TPRs exists or not.

In order to isolate a protein to be bound to this HDART, a yeast two-hybrid system was used. More particularly, a yeast MATCHMAKER two-hybrid analyzing kit (Clontech) was used for the isolation. The ORF full length of HDART was inserted so that the Ga14 DNA binding domain and a reading frame were coincident with each other within pAS-1 vector (Clontech). This bait plasmid was transformed into Saccharomyces cerevisiae Y190 along with pACT2 prey plasmid whose HeLa cDNA (Clontech) had been sub-cloned. The screening of the clones into which both plasmids were introduced was carried out according to the protocol attached to the kit. In this way, the results of cloning at about 1 × 10⁷ lead to isolation of several clones. The analysis of these clones revealed that one coincided with Skip.

The interaction between HDART and Skip inside the mammal cells was confirmed according to the immunoprecipitation analysis. For the confirmation, the Flag-Skip expression vector expressing the Flag-Skip fused protein and the GFP-HDART expression protein (FIG. 4A) expressing the GFP-HDART fused protein were transfected into HEK293 cells by use of Effectene kit (QIAGEN). It will be noted that a control experiment was conducted such that the Flag-Skip expression vector alone and the GFP-HDART expression vector were, respectively, transfected into cells of the same type under the same condition.

24 hours after the transfection, the cells were lysized on ice for 30 minutes in a Nonidet P-40 buffer solution (50 mM of tris HCL (pH 7.6), 150 mM of NaCl, 5 mM of EDTA, 0.5% of Nonidet P-40 and 1 mM of PMSF) containing 100 µl of a protease inhibitory substance cocktail (Sigma #p8340) to prepare a cell extract (1 mg). This extract was incubated for 30 minutes at 4°C along with 40 µl of protein A/G sepharose for preliminary clarification. Next, the thus clarified supernatant extract was further incubated for 1 hour along with an anti-Flag antibody or a negative control mouse IgG antibody (2 µg), followed by precipitation for 30 minutes by use of 40 µl of protein A/G sepharose beads. The resulting immunoprecipitate was washed four times with a Nonidet P-40 buffer solution. The bound protein was dissolved out from A/G sepharose beads in an SDS loading buffer to develop the eluate with SDS-PAGE. After the development, transcription on a membrane was carried out, followed immunoblotting of the membrane in a usual manner. For an antibody of this immunoblotting, there were used anti-FLAG antibody M2 (Sigma) and anti-GFP monoclonal antibody cone 1E4 (MBL).

The results of the immunoprecipitation analysis are shown in FIG. 4A. In the figure, the upper two panels show the results of expression of the respective proteins, and the lower two panels show those of the immunoprecipitates. A shown in FIG. 4A, only when both composite proteins are expressed, the GFP-HDART protein was immunoprecipitated by means of the anti-Flag antibody along with the Flag-Skip fused protein (lane 2). Under conditions where no FLAG-skip is expressed, no precipitation of GFP-HDART with the anti-FLAG antibody was observed (lane 1), no precipitation was likewise observed in the case using the negative control antibody (lane 3). These results suggest that HDART and Skip specifically interact in vivo.

Further, the interaction of endogenous HDART and externally introduced Skip was analyzed. A Flag-skip expression vector or a Flag-lucipherase expression vector was transfected into 293 cells (i.e. cells highly expressed with HDART), followed by preparation of a cell extract 24 hours after the tranfection in the same manner as set out hereinbefore. This cell extract was incubated with an anti-Flag antibody and immunoprecipitated. The immunocomplex or cell extract prior to the immunoprecipitation was developed with SDS-PAGE, followed by transcription on a membrane. The same membrane was immunoblotted by use of an anti-HDART antibody or an anti-Flag antibody. The results are shown in FIG. 4B. It should be noted that in FIG. 4B, the upper panel shows the results of immunoblotting of the cell extract with the anti-HDART antibody, the central panel shows the results of immunoblotting with the anti-Flag antibody after immunoprecipitation with the anti-Flag antibody, and the lower panel show the results of immunoblotting with the anti-HDART antibody after immunoprecipitation using the anti-Flag antibody.

As shown in FIG. 4B, HDART was co-precipitated by means of the anti-Flag antibody only under conditions of expression of Flag-Skip (lane 1 of FIG. 4B), and no coprecipitation was observed under conditions the Flag-Luc protein was expressed (lane 2) and with the parent 293 clone where nothing was transfected (lane 3).

### [Example 3] Binding region of HDART and Skip

For mapping of regions on the Skip taking part in the interaction with HDART, a series of deleted mutations where various regions on Skip (N-terminus region (codon 1-220), nuclear hormone binding region (NHR binding, codon 221-388), and tran-activation region (TA, codon 438-536) were deleted, and the interaction between HDART and mutated Skip was analyzed according to the yeast two-hybrid analysis using Ga14DBD-HDART set forth in Example 2. The results of the analysis are shown in FIG. 5A. The sign "+" indicated at the right of FIG. 5A indicates the observation of the interaction by the filter lift analysis of β-galactosidase activity, and the number of "+" indicates the relative intensity of the interaction. The sign "-" indicates no detection of the interaction.

As shown in FIG. 5A, we discovered that two different regions takes part in the interaction with HDART. Their regions include one within 97-119 residues and the other within 220-437 residues. It was shown that the trans-activation region of Skip scarcely takes part in the binding activity with HDART. A similar approach was carried out for analyzing the region taking part in the interaction with Skip on HDART. More particularly, various deleted allelic mutants of Ga14DBD-HDART were prepared to analyze the galactosidase upon interaction thereof with Ga14AD-Skip. The results of the analysis are shown in FIG. 5B. It was proved that the N-terminus region including four TPRs (1-179 residues) ensures the interaction with Skip to a full extent (FIG. 5B). Accordingly, HDART is able to directly interact with Skip via the four TPR regions of the N-terminus.

### [Example 4] Transcription inhibition, with HDART, of the gene ascribed to nuclear receptor

Since it is shown in the above example that HDART is able to interact with Skip, a functional role of HDART against the transcription route ascribed to a nuclear receptor was analyzed. Initially, the action of HDART on the transcription control with retinoic acid receptor was analyzed. For this purpose, the CAT analysis was made in such a way that RAR (retionic acid receptor) incorporating a thymidine kinase minimum promoter (pTREpal-tata) and CAT gene therein is used downstream of a retinoid response element is employed.

More particularly, pcDNA3-HDART capable of steadily expressing HDART was transfected into HepG2 cells by use of Effectene kit (QIAGEN), simultaneously with RAR reporter plasmid. It is to be noted that the pcDNA3-HDART expression vector was introduced in different amounts of 0, 0.5 and 1.0 µg, and the amounts of DNA in the respective transfections were made equally at 1 µg under control of a vacant vector of pcDNA. After the transfection, growth was made in the presence or absence of ATRA (10⁻⁸ M) to measure the CAT activity at that time. The results of the measurement (FIG. 6) are indicated in terms of values corrected on the basis of the CAT activity obtained after introduction of 1.0 µg of average of the results of three experiments and a standard deviation in terms of an error bar are also shown.

As shown in FIG. 6A, with the cells introduced with the vacant vector (pcDNA), the CAT activity was increased, by the action of ATRA, to five times that not introduced. However, this ATRA-induced cat activity was inhibited depending on the concentration of HDART.

Furthermore, the action of HDART on the transcription control of glucocorticoid receptor (GR) was analyzed by use of GR reporter plasmid within GR positive Hela cells. The transcription activity of a glucocorticoid response promoter was carried out in the same manner as in the experiment for the retinoic acid receptor except for the use of Hela cells and 10⁻⁸ M of dexamethasone. The results of the measurement are shown in the same manner as shown above.

The co-expression of HDART was repressed to a similar extent as observed with the RAR (retinoic acid receptor) induced trans-activation and thus, the activation of the reporter gene responding to glucocorticoid was inhibited (FIG. 6B). These results show that HDART selectively represses the transcription activated by means of the nuclear receptor.

### [Example 5] Localization of HDART in cell nuclei

Since it is shown that HDART directly takes part in the transcription control, it is presumed that HDART is localized in nuclei of cells. Based on this, a polyclonal antibody against a HDART combined protein was prepared to analyze the localization of HDART within cells by an immunofluorescent experiment.

The preparation of the polyclonal antibody was produced in Escherichia coli as His-HDART (amino acid residues 296-431) fused protein, followed by purification with a Ni-NTA resin (QIAGEN) showing affinity for His. Next, the His-HDART protein was immunized to a rabbit, and the resulting anti-HDART antiserum was further purified according to affinity chromatography using Prot0n kit 1 (MPS). The thus purified rabbit anti-HDART antibody was incubated with Hela cells inherently holding HDART therein, followed by further incubation with PE (phycoerythrin) fused rabbit antibody for carrying out immunofluorescent staining. It will be noted that for a control test, a similar procedure was repeated using rabbit serum prior to immunity in place of the rabbit anti-HDART antibody. In addition, in order to clarify the location of nucleus, DAPI staining was also carried out.

As shown in FIG. 7A, the immunolfuorescent stained image using the anti-HDRT antibody was in coincidence with DAPI stained image. On the other hand, no nucleus was stained for the preimmune serum. From these results, it was shown that HDART was predominantly localized within nuclei of the Hela cells (FIG. 7).

The location of HDART in vital cells was analyzed. A GFP or GFP-HDART expression vector was transfected into Hela cells. After 24 hours, fluorescence with GFP was detected. As to the GFP-HDART cells, incubation was performed using Hoechest 33342 dye so as to visualize the nuclei.

As shown in FIG. 7B, with the cells transfected with GFP-HDART vector, it was confirmed that the nuclei visualized with Hoechest 33342 dye (upper right panel) emits fluorescence with GFP (left upper panel). Especially, GFP-HDART showed a pattern partly similar to the hitherto reported nuclear speckle pattern (17) of the Skip molecule. Similar results were also observed with respect to HT1080 cells and 293 cells (not shown).

### [Example 6] Autonomous Repression function caused by HDART

Assuming that HDART takes part in more direct inhibition, it is expected that it has an automonous repression function. In order to confirm the above expectation, the full-length HDART cDNA for combining HDART with DNA was fused to Gal4 DNA binding region (GAL4DBD: a region having only a binding region with GAL4DNA without a transcription control region) to analyze whether the transcription from the GAL4A promoter is controllable within NIH3T3 cells.

More particularly, Ga14 DBD-HDART plasmid capable of expressing a fused protein of the HDART full-length protein and Ga14 DBD was transfected into NIH3T3 cells introduced with Ga14 reporter plasmid (pGal4-Luciferase) beforehand in different amounts (0, 0.1, 0.3 and 0.5 µg). It should be noted that in order to make a uniform total amount of DNA used for the transfection, the amount of an expression vector was controlled to 0.5 µg using a vacant vector of Gal4 DBD in individual transfections. 24 hours after the transfection, the expression activity (Luciferase activity) of reporter genes from the promoter was analyzed. The luciferase activity of the respective samples is indicated as a corrected value based on the reference (100%) of luciferase activity obtained by introduction of a vacant vector alone (FIG. 8). It will be noted that the results of the analysis were indicated as an average of the results of three experiments, and a standard deviation is indicated as an error bar (FIG. 8A). Similar analyses were made using a different type of cell of U-20S cell with use of Gal4 DBD-HDART (in an amount of 0 or 0.5 µg) (FIG. 8B).

HDART significantly inhibits the promoter activity within the NIH-3T3 cells in dependence of the concentration thereof, and lower the expression of luciferase by 80% at the highest concentration (0.5 µg) (FIG. 8A). Similar results were observed with the case of the U-20S cell (FIG. 8B). From these results, it was shown that HDART has the autonomous transcription inhibitory action by itself.

With HDART having no GAL4 DNA binding region, no expression inhibition of luciferase was observed (not shown). From this, it will be seen that HDART has no binding activity of the promoter region to DNA.

### [Example 7] Suppressing mechanism ascribed to HDART

It has been reported that acute transcription is controlled by the degrees of activation of HAT (histone acetylase) and acetylization of core histone through a mechanism in which inhibition with HDAC (histone decetylase) takes part. In order elucidate the genetic repression mechanism caused by HDART, whether or not this function of HDART is shown through the formation of a complex with HDAC was checked according to an immunoprecipitation analysis using Flag-HDAC expression vector.

Flag-HDACs expression vectors capable of expressing different types of HDAC (1, 3, 4 or 6) and a GFP-HDART expression vector were, respectively, co-transfected with 293 cells in the same manner as in Example 2 to provide cell extracts 24 hours after the transfection. The respective cell extracts were incubated with an anti-Flag antibody, followed by immunoprecipitation. The resulting immunoprecipitate was isolated with SDS-PAGE and an isolated pattern was transcripted on a membrane, followed by immunoblotting with use of an anti-Flag antibody or anti-GFP antibody. For reference, in order to confirm the expression of GFP-HDART protein in the respective cells, individual samples prior to the immunoprecipitation were similarly developed with SDS-PAGE to conduct immunoblotting with use of an anti-GFP antibody. It will be noted that in FIG. 9A, the upper panel shows the results of the expression of the GFP-HDART protein prior to the immunoprecipitation, the central panel shows the results of the immunoblotting of the immunoprecipitate with the anti-Flag antibody, and the lower panel shows the results of the immunoblotting of the immunoprecipitate with the anti-GFP antibody. The kinds of HDAC's from the left side of the figure are as follows: lane 1; HDAC1, lane 3; HDAC3, lane 4; HDAC4, and lane 5; HDAC6. It will be noted that lane 2 is for the sample expressed with GFP-HDART alone.

As shown in FIG.9A, with a 293 cell-derived extract wherein the Flag-HDAC expression vector and the GFP-HDART expression vector were co-transfected, it was confirmed that GFP-HDART was immunoprecipitated with the anti-FLAG antibody (lower panel, lanes 1, 3, 4, 5 from the left). However, no precipitation of GFP-HDART was observed in the absence of FLAG-HDAC (a line into which no Flag-HDAC expression vector was introduced) when the anti-FLAG antibody was added (lower panel, lane 2). Accordingly, it was proven that the precipitation of GFP-HDART with the anti-FLAG antibody depends on the specific interaction between Flag-HDAC and GFP-DART. Moreover, it was also shown that HDART exhibits interaction with both types of HDAC's including type I (HDAC's 1 and 3) and type II (HDAC's 4 and 6).

Further, the direct interaction between HDART and HDAC3 was checked by GST pulldown analysis. The GST pulldown analysis was fundamentally carried out according to a known procedure (Tzamarias, D., and Struhl, K. (1995) Genes Dev 9(7), 821-31.). Using TNT (Registered Trademark) in vitro transcription/translation system (Promega), the in vitro translation of HDAC3 was made in the presence of ³⁵S-methionine. GST protein or GST-HDART fused protein was, respectively, expressed within Escherichia coli, followed by purification in a GST binding buffer solution (50 mM tris-HCl, 200 mM LiCl, 0.5% NP40, 5 mM EDTA, 1 mM PMSF) by use of glutachion sepharose. A binding reaction solution containing GST-HDART fused protein or control GST protein (about 1 µg) and ³⁵S-radio-labeled in vitro translation product (10 µl) in 1 ml of the GST binding buffer solution was prepared. This reaction solution was incubated at 4°C for one hour while shaking, after which the resulting the sepharose-GST protein complex was washed five times with the GST binding buffer solution. The protein bound with the GST protein was dissolved out by boiling in a sodium dodecylsulfate (SDS) containing sample buffer solution, followed by isolation with SDS-polyacrylamide gel electrophoresis. It was confirmed by Coomassie brilliant blue staining that the GST fused protein was equally phoresized, and ³⁵S-radio-labeled HDAC was detected according to autoradiography.

As shown in FIG. 9B, the In vitro translated HDAC3 did not bind to the mere GST protein and could not be pulled down. However, when using the GST-HDART fused protein, to be pulled down was shown (FIG. 9B). Although not shown in the figure, similar results were obtained for HDAC1. From this, it was suggested that the interaction between the HDART and HDAC was direct in nature.

In order to analyze the influence of the transcription inhibition action of HDART on the deactylization activity of HDAC, CAT reporter analysis was carried out in the manner as in Example 4 in the presence of tricostatin A (TSA) that specifically inhibits HDAC (FIGS. 10C and D). In this example, however, a given amount of pcDNA3-HDART expression vector (1.□g) ("HDART+" in FIG. 10) and 100 nM of TSA or 1 mA of sodium butyrate was added to along with a ligand (ATRA or dexamethazone).

As shown in FIGS. 10C, D, the expression of the reporter gene from the promoter corresponding to the ligand alone increased, in which when HDART was expressed, the expression activity was suppressed. When triconstatin A was further added, the expression repression with HDART was fully neutralized. The same results were also observed in the case where another type of histone deacetylase inhibitor and sodium butyrate (Buty) were added. These results backed up that the transcription inhibition with HDART is shown via the deacetylase activity.

### [Example 8] Active repression of unliganded receptor with HDART-Skip interaction

RAR and TR inhibit the genetic activity in the absence of a ligand in vivo (Baniahmad, A., Kohne, A. C., and Renkawitz, R. (1992) Embo J 11(3), 1015-23) and in vitro (Fondell, J. D., Roy, A. L., and Roeder, R. G. (1993) Genes Dev 7(7B), 1400-10). From this, this is called active repression. Skip interacts with NHR independently of ligand (MacDonald, P. N., Baudino, T. A., Toumaru, H., Dowd, D. R., and Zhang, C. (2001) Steroids 66 (3-5), 171-6). In view of these repressing effects and the physiological involvement with Skip, there has been suggested the possibility that the HDART-Skip composite body exists on the unliganded receptor or the possibility that the interaction takes part in the active repression of receptor. In order to clarify these possibilities, a dominant negative line of HDART is excessively expressed to check the influence on the transcription ascribed to RAR. As shown in Example 2, the four TRPs (N4TPR) at the N terminus of HDART belongs to the Skip binding region, so that it is assumed that when the expression in this region inhibits the interaction between the internal HDART and Skip is suppressed to inhibit the natural function of HDART for functioning as a dominant negative. In this sense, the N4TPR was used as a candidate for dominant negative in this example.

Flag-Skip and N4TPR tagged with GFP or GFP were transfected into 293 cells. 24 hours after the tranfection, a cell extract was prepared, followed by immunoprecipitation with an anti-Flag antibody. The resultant immunoprecipitate was isolated on SDS-PAGE. To confirm the expression of the endogeneous HDART prior to the immunoprecipitation, a cell extract prior to the immunoprecipitation was likewise isolated with SDS-PAGE. The pattern after the isolation was copied on a membrane. The expression of Flag-Skip was detected using an anti-Flag antibody, the expression of GFP and GFP-N4TPR detected using an anti-GFP antibody, and the expression of the internal HDART detected using an anti-HDART antibody, respectively (FIG. 11A). It will be noted that in FIG. 11A, the lower three panels, respectively, show precipitated Skip (top of the lower panels), endogenous HDART (middle of the lower panels), and N4TPR (bottom of the lower panels), and one top panel shows the expression of an endogenous HDART protein prior to the immunoprecipitation.

As shown in FIG. 11A, the expression of N4TPR lead to a more reduced amount (lower center panel, lane 2) of HDART co-precipitated with Skip in comparison with a control where no N4TPR was expressed (GFP alone, lane 1). On the other hand, excess expression of N4TPR contributes to increasing the interaction between N4TPR and Skip, with a remarkable increase in amount of the co-precipitated Skip (lane 2, lower bottom panel). The expression of the control protein (GFP) exhibits no influence on the interaction between HDART and Skip (lane 1). The results show that N4TPR acts as a dominant negative protein which interacts with Skip in place of HDART.

Next, the influence of the excess expression of N4TPR on the transcription from RAR or a glucocorticoid responsible promoter was checked. It will be noted that this checking was made in the same manner as in the reporter analysis set out in Example 4, but using GFP-N4TPR expression vector (0, 0.3 and 0.5 µg).

The results are as shown in FIGS. 11B, C wherein the transcription activity from the RAR and glucocorticoid responsible promoter was increased to an extent (gray column) of transcription activity derived in the presence of a ligand by permitting the expression while limiting N4TPR in the absence of a ligand. At the highest expression level (amount: 0.5 µg) of N4TPR, the transcription activity in the absence of a ligand was strongly increased (to about 20 times). These results suggest that HDART is necessary for the active repression of nuclear hormone receptors such as RAR, glucocorticoid responsible promoter and the like.

### [Example 9] Inhibition, by excess expression of HDART, of differentiation of rhabdomyosarcoma cell line into muscular tissue derived from retinoic acid

It is known that ATRA (all-trans retinoic acid) is an important inducer for tumor cell differentiation (20-22). The human rhabdomyosarcoma cell line MM-1-19-P is constituted mainly of polygonal cells and finally differentiated into myotubular cytomegalic cells provided with retionic acid. In order to clarify the physiological role of HDART in he reaction ascribed to nuclear hormone receptors, the influence of the expression with HDART on the differentiation of MM-1-19-P caused by ATRA was analyzed.

MM-1-19-P cells were planted on a 100 mM Petri dish, followed by co-transfection of the cells with 0.4 µg of pGFP vector and 2 µg of pcDNA3 (vacant vector) or pcDMA3-HDART (HDART expression vector). 24 hours after the transfection, the medium was changed with an ATRA-containing (2 µM) or ATRA-free fresh one. After induction over 24 hour, at the time when the cells was changed into elongated spindle cells exhibiting myotubular cytomegalic cells, the cells were assessed as having been morphologically differentiated. All experiments were repeated four times, and the positive for GFP was determined with the number of the assessed cells being counted. The results are shown in FIG. 12. It will be noted that in FIG. 12, the standard microphotographs of green fluorescence of GFP are shown in panels of FIGS. 12A, 12B, 12C and 12D, and the phase differential microphotographs are shown in panels of FIGS. 12E and 12F, respectively. In the figure, 12A indicates ATRA non-treated, vacant vector-introduced cells, 12B indicates ATRA treated, vacant vector-introduced cells, 12C indicates ATRA-non-treated, HDART expression vector-introduced cells, 12D indicates ATRA-treated, HDART expression vector-introduced cells, 12E indicates cells under the same conditions as in 12C above, and 12F indicates cells under the same conditions as in 12D above. In addition, the black arrow in the panel of FIG. 12F indicates unspecialized GFP positive cells, and white arrow indicates differentiated cells. In FIG. 12G, the graph shows a percentage of morphologically differentiated cells in the GFP positive cells as an average of the results of four independent experiments, and a standard deviation is indicated in terms of error bar (P<1% between a vacant vector and an ATRA (+) and HDART expression vector, t test of student).

In the respective experiments, the number of GFP positive cells ranged 30 to 70. With the cells treated with the vacant vector-introduced ATRA, the number of GFP positive cells was found to be less than 30% because of the ATRA-induced cytotoxicity. With the HDART expression vector-introduced cells, the numbers of GFP positive cells were same for the ATRA treated group and non-treated group.

Most of the vacant-introduced cells were differentiated into muscular tissue as shown by the appearance of the myotubular cytomegalic cells (FIGS. 12B, FIG. 12G). With the vacant vector-introduced cells, the degrees of change in phenotype caused by the ATRA treatment were same for both positive and negative cells. However, with the HDART-introduced cells, little change in the phenotype appeared when the GFP positive cells were subjected to ATRA treatment (black arrow in FIG. 12D, FIG. 12G). On the other hand, with GFP negative cells, characteristic mytotubular cytomegalic cells were observed (white arrow in FIG. 12F). These results show that the expression of HDART represses the differentiation with retinoic acid. The results are coincident with those results of repressing the transcription activity RAR with HDART in the reporter analyses. These results reveal that HDART serves at least as a co-repressor of physiological transcription in retinoic acid receptor.

### Industrial Applicability

As stated hereinabove, the transcription repressor of the invention autonomously represses transcription, particularly, the transcription of nuclear receptors, and thus acts on desired transcription systems and can be used for the purpose of repressing transcription of the transcription systems. Moreover, the transcription repressor of the invention has the bindability on HDAC and is able to show the transcription repressing ability via the histone deacetylization activity of this HDAC. Accordingly, the HDAC is recruited by use of the transcription repressor of the invention thereby repressing transcription by the action of the HDAC. This action of the transcription repressor of the invention is applicable to diseases resulting, for example, from increased transcription of nuclear receptors. The transcription repressor of the invention is useful as a curing medicine for these diseases.

The dominant negative peptide of the transcription repressor functions as a transcription activity factor. Accordingly, this dominant negative peptide can be used to promote the transcription. This dominant negative peptide also acts for transcription of nuclear receptors and reversely promote the transcription. Hence, the peptide is useful as a transcription promoting substance for nuclear receptor. Especially, the four TPRs at the N terminus side of HDART (N4TPR) has higher transcription activity of retinoic acid receptor than ATRA, and thus, the peptide of the invention is applicable to as a curing medicine in place of ATRA or along with ATRA in treatment of diseases where ATRA is currently employed (e.g. in a differentiation-inducing therapy or the like).

## Claims

1. A dominant negative HDART peptide having transcription activating activity which:
consists of amino acids 1 to 179 of SEQ ID No 2; or
is a peptide encoded by a DNA consisting of a base sequence of bases 1 to 537 of SEQ ID No 1; or
consists of amino acids 1 to 179 of SEQ ID No 2 having one or more
substitutions, deletions, insertions and/or additions to the sequence,
for use in activating the transcription of nuclear receptors.

2. The peptide according to claim 1 wherein the nuclear receptor is selected from the group consisting of: retinoic acid; glucocorticoid receptor; retinoin X receptor, vitamin D receptor, androgen receptor, esterogen and tiroid hormone receptor.

3. A peptide according to claim 1 for use as a medicament.

4. The peptide according to claim 3 for use in treating a disease selected from the group consisting of the following: leukaemia; hepatic cell carcinoma; ovarian cancer; thyroid cancer; skin cancer; pancreatic cancer.

5. An expression vector comprising:
(A) a DNA encoding a peptide consisting of an amino acid sequence from position 1 to 179 in SEQ ID No 2; or
(B) a DNA consisting of a base sequence of from 1 to 537 bases in SEQ ID No 1; or
(C) a DNA encoding a peptide consisting of an amino acid sequence from position 1 to 179 in SEQ ID No 2 having one or more substitutions, deletions, insertions and/or additions to the sequence and having transcription activating activity, for use in treating a disease selected from the group consisting of the following: leukaemia; hepatic cell carcinoma; ovarian cancer; thyroid cancer; skin cancer; pancreatic cancer.

6. A host cell comprising an expression vector as described in claim 5 for use in treating a disease selected from the group consisting of the following: leukaemia; hepatic cell carcinoma; ovarian cancer; thyroid cancer; skin cancer; pancreatic cancer.

7. Use of a peptide as described in claim 1 or an expression vector as described in claim 5 for activating transcription *in vitro.*

## Patentansprüche

1. Dominant-negatives HDART-Peptid mit einer Transkriptionsaktivierungsaktivität, das
aus Aminosäuren 1 bis 179 der SEQ I17. No.2 besteht, oder
ein Peptid ist, das durch eine aus einer Basensequenz von Basen 1 bis 537 der SEQ ID No.1 bestehenden DNA codiert ist, oder
aus Aminosäuren 1 bis 179 der SEQ ID No.2 besteht, die eine oder mehrere Substitutionen, Entfernungen, Einfügungen und/oder Hinzufügungen bei der Sequenz aufweisen,
zur Benutzung bei Aktivierung der Transkription von nucleären Rezeptoren.

2. Peptid nach Anspruch 1, wobei der nucleäre Rezeptor aus der Gruppe bestehend aus: Retinsäure, Glucocorticoidrezeptor, Retinoin-X-Rezeptor, Vitamin-D-Rezeptor, Androgenrezeptor, Estrogen- und Thyroidhormonrezeptor ausgewählt ist.

3. Peptid nach Anspruch 1 zur Benutzung als ein Medikament.

4. Peptid nach Anspruch 3 zur Benutzung bei Behandlung einer Krankheit, ausgewählt aus der aus dem Folgenden bestehenden Gruppe: Leukämie, Leberzellkarzinom, Eierstockkrebs, Schilddrüsenkrebs, Hautkrebs, Pankreaskrebs.

5. Expressionsvektor, aufweisend:
(A) eine DNA, die ein Peptid, bestehend aus einer Aminosäurensequenz von Position 1 bis 179 in der SEQ ID No.2, codiert, oder
(B) eine DNA, bestehend aus einer Basensequenz von 1 bis 537 Basen in der SEQ ID No. 1, oder
(C) eine DNA, die ein Peptid codiert, das besteht aus einer Aminosäurensequenz von Position 1 bis 179 in der SEQ ID Nr.2, die eine oder mehrere Substitutionen, Entfernungen, Einfügungen und/oder Hinzufügungen bei der Sequenz aufweist und eine Transkriptionsaktivierungsaktivität aufweist, zur Benutzung bei Behandlung einer Krankheit, ausgewählt aus der aus dem Folgenden bestehenden Gruppe: Leukämie, Leberzellkarzinom, Eierstockkrebs, Schilddrüsenkrebs, Pankreaskrebs.

6. Wirtszelle, aufweisend einen Expressionsvektor wie in Anspruch 5 beschrieben zur Benutzung bei Behandlung einer Krankheit, ausgewählt aus der aus dem Folgenden bestehenden Gruppe: Leukämie, Leberzellkarzinom, Eierstockkrebs, Schilddrüsenkrebs, Hautkrebs, Pankreaskrebs.

7. Benutzung eines Peptids wie in Anspruch 1 beschrieben oder eines Expressionsvektors wie in Anspruch 5 beschrieben zur Aktivierung einer Transkription *in vitro.*

## Revendications

1. Peptide HDART dominant négatif ayant une activité d'activation de la transcription qui :
consiste en les acides aminés 1 à 179 de SEQ ID NO: 2 ; ou
est un peptide codé par un ADN consistant en une séquence de bases constituée des bases 1 à 537 de SEQ ID NO: 1 ; ou
consiste en les acides aminés 1 à 179 de SEQ ID NO: 2 avec une ou plusieurs
substitutions, délétions, insertions et/ou additions à la séquence,
pour une utilisation dans l'activation de la transcription de récepteurs nucléaires.

2. Peptide selon la revendication 1, où le récepteur nucléaire est sélectionné dans le groupe consistant en : l'acide rétinoique ; le récepteur des glucocorticoïdes; le récepteur X des rétinoïdes ; le récepteur de la vitamine D ; le récepteur des androgènes, le récepteur des oestrogènes et des hormones thyroïdiennes.

3. Peptide selon la revendication 1, destiné à être utilisé en tant que médicament.

4. Peptide selon la revendication 3, destiné à être utilisé pour traiter une maladie sélectionnée dans le groupe consistant en les maladies suivantes : une leucémie; un carcinome hépatocellulaire; le cancer de l'ovaire ; le cancer de la thyroïde ; le cancer de la peau ; le cancer du pancréas.

5. Vecteur d'expression comprenant :
(A) un ADN codant pour un peptide consistant en une séquence d'acides aminés comprise entre la position 1 et 179 dans SEQ ID NO: 2 ; ou
(B) un ADN consistant en une séquence de bases comprise entre les bases 1 et 537 dans SEQ ID NO: 1 ; ou
(C) un ADN codant pour un peptide consistant en une séquence d'acides aminés comprise entre la position 1 et 179 dans SEQ ID NO: 2 avec une ou plusieurs substitutions, délétions, insertions et/ou additions à la séquence et ayant une activité d'activation de la transcription,
pour une utilisation dans le traitement d'une maladie sélectionnée dans le groupe consistant en les maladies suivantes : une leucémie ; un carcinome hépatocellulaire ; le cancer de l'ovaire ; le cancer de la thyroïde ; le cancer de la peau ; le cancer du pancréas.

6. Cellule hôte comprenant un vecteur d'expression tel que décrit dans la revendication 5, pour une utilisation dans le traitement d'une maladie sélectionnée dans le groupe consistant en les maladies suivantes : une leucémie ; un carcinome hépatocellulaire ; le cancer de l'ovaire ; le cancer de la thyroïde ; le cancer de la peau ; le cancer du pancréas.

7. Utilisation d'un peptide tel que décrit dans la revendication 1 ou d'un vecteur d'expression tel que décrit dans la revendication 5 pour activer la transcription *in vitro.*
